(19) **Europäisches Patentamt / European Patent Office / Office européen des brevets**

(11) **EP 1 656 171 B1**

(12) **EUROPEAN PATENT SPECIFICATION**

(45) Date of publication and mention
of the grant of the patent:
**09.06.2010 Bulletin 2010/23**

(51) Int Cl.:
**A61M 11/04** (2006.01)

(21) Application number: **04780193.1**

(22) Date of filing: **04.08.2004**

(86) International application number:
**PCT/US2004/025313**

(87) International publication number:
**WO 2005/016421 (24.02.2005 Gazette 2005/08)**

(54) **SUBSTRATES FOR DRUG DELIVERY DEVICE AND METHODS OF PREPARING**

SUBSTRATE FÜR EINE MEDIKAMENTENVERABREICHUNGSVORRICHTUNG UND VERFAHREN ZUR BEREITUNG

SUBSTRATS POUR DISPOSITIF D'ADMINISTRATION DE MEDICAMENTS ET PROCEDES DE PREPARATION

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **04.08.2003 US 492630 P**

(43) Date of publication of application:
**17.05.2006 Bulletin 2006/20**

(73) Proprietor: **Alexza Pharmaceuticals, Inc.**
**Mountain View, CA 94043 (US)**

(72) Inventors:
• **BENNETT, Bryson**
**Mountain View, CA 94043-4551 (US)**
• **HALE, Ron, L.**
**Woodside, CA 94062 (US)**
• **LU, Amy, T.**
**Los Altos, CA 94024 (US)**

• **MYERS, Daniel, J.**
**Mountain View, CA 94043 (US)**
• **RABINOWITZ, Joshua, D.**
**Princeton, NJ 08542 (US)**
• **SHARMA, Krishnamohan**
**Santa Clara, CA 95054 (US)**
• **WENSLEY, Martin, J.**
**San Francisco, CA 94107 (US)**

(74) Representative: **Clegg, Richard Ian et al**
**Mewburn Ellis LLP**
**33 Gutter Lane**
**London**
**EC2V 8AS (GB)**

(56) References cited:
**EP-A- 1 222 938      WO-A-94/09842**
**WO-A-02/094232      WO-A-02/098389**
**US-A- 1 514 682      US-A1- 2003 106 551**

**Description**

**FIELD OF THE INVENTION**

The invention relates generally to the field of devices for administration of pharmaceutically-adive agents (*e.g.*, drugs). More specifically, the invention relates to a drug-supply assembly for incorporation in an inhalation device for use in production of drug-aerosol particles.

**BACKGROUND**

Traditionally, inhalation therapy has played a relatively minor role in the administration of therapeutic agents when compared to more traditional drug administration routes of oral delivery and delivery via injection. Due to drawbacks associated with traditional routes of administration, including slow onset, poor patient compliance, inconvenience, and/or discomfort, alternative administration routes have been sought. Pulmonary delivery is one such alternative administration route which can offer several advantages over the more traditional routes. These advantages include rapid onset, the convenience of patient self-administration, the potential for reduced drug side-effects, ease of delivery by inhalation, the elimination of needles, and the like. Many preclinical and clinical studies with inhaled compounds have demonstrated that efficacy can be achieved both within the lungs and systemically.

However, despite such results, the role of inhalation therapy in the health care field has remained limited mainly to treatment of asthma, in part due to a set of problems unique to the development of inhalable drug formulations, especially formulations for systemic delivery by inhalation. Metered dose inhaler formulations involve a pressurized propellant, which is frequently a danger to the environment, and generally produce aerosol particle sizes undesirably large for systemic delivery by inhalation. Furthermore, the high speed at which the pressurized particles are released from metered dose inhalers makes the deposition of the particles undesirably dependent on the precise timing and rate of patient inhalation. While solving some of the problems with metered dose inhalers, dry powder formulations are prone to aggregation and low flowability phenomena which considerably diminish the efficiency of dry powder-based inhalation therapies. Such problems are particularly severe for dry powders having a small enough aerosol particle size as to be optimal for deep lung delivery, as difficulty of particle dispersion increases as particle size decreases. Thus, excipients are needed to produce powders that can be dispersed. Liquid aerosol formations similarly involve non-drug constituents, i.e. the solvent, as well as preservatives to stabilize the drug in the solvent. Dispersion of liquids generally involves complex and cumbersome devices and is effective for only solutions with specific physical properties, *e.g.* viscosity. Such solutions cannot be produced for many drugs due to the solubility properties of the drug. In addition, these added excipients, solvents, propellants, etc., impact the purity of the resultant delivered drug. Purity is a critical issue that must be address for delivery of a drug to humans.

Volatilization of a drug to form an aerosol while addressing many of the above mentioned problems subjects the drug to potential chemical degradation via thermal, oxidative, and/or other means. Volatilization can also impact the purity of the drug being delivered.

Thus, there remains a need in the art for devices capable of producing a drug aerosol for delivery by, for example, inhalation or topical application and, in particular, devices that create highly pure aerosols that do not require added excipients to improve flowability and prevent aggregation, and/or solvents, propellants, or drug solubility to disperse the drug. WO 02/098389 A describes an exemplary drug supply assembly having a thin stainless steel foil on to which a dose of a compound is deposited. The compound may be coated as a film of between 10μm and 10nm.

**SUMMARY**

The invention provides a drug-supply assembly comprising a metal substrate and a film comprising a drug compound. The exterior surface of the metal is metal oxide enriched. The metal oxide enriched surface can be the result of heat or chemical treatment of the substrate. Alternatively, the metal oxide enriched surface can be a heterologous layer of material that is applied to the substrate. Exemplary metal substrates include steel, stainless steel, aluminum, titanium and copper. The metals substrates can be treated by heat or chemicals to generate an exterior surface comprising, for example, metal oxides such as iron oxide, chromium oxide, zirconium oxide, aluminum oxide, silicon oxide, silicon carbide, or a combination thereof. Alternatively, the substrate can be coated with a oxidation resistant material such as, for example, zirconium oxide, silicon oxide, aluminum oxide, aluminum nitride, and/or silicon carbide.

In another aspect, the invention includes a method of preparing the above described drug-supply assembly for use in an aerosol device, comprising treating a substrate with heat and/or a chemical to generate an oxidation resistant exterior surface. In another aspect, the substrate is coated with an oxidation resistant material to generate an oxidation resistant exterior surface. The exterior surface is then coated with a film comprising a drug.

There is also disclosed a method of increasing the purity of drug condensation particles in a condensation drug aerosol that is produced by substantially vaporizing and condensing a film comprising a drug on a substrate comprising substantially vaporizing a drug composition on a modified metal substrate and condensing the vapor to form drug particles, said method however does not belong to the invention.

**BRIEF DESCRIPTION OF THE DRAWINGS**

Figs. 1A-1B are cross-sectional views of general embodiments of a drug-supply assembly in accordance with the invention.

Fig. 2A is a perspective view of a drug-delivery device that incorporates a drug-supply assembly.

Fig. 2B shows another drug-delivery device that incorporates a drug-supply assembly, where the device components are shown in unassembled form.

Figs. 3A-3E are high speed photographs showing the generation of aerosol particles from a drug-supply device comprising a drug-supply assembly.

Figs. 4A-4B are plots of substrate temperature increase, measured in still air with a thin thermocouple (Omega, Model CO2-K), as a function of time. The substrate in Fig. 4A was heated resistively by connection to a capacitor charged to 13.5 Volts (lower line), 15 Volts (middle line), and 16 Volts (upper line); the substrate in Fig. 4B was heated resistively by discharge of a capacitor at 16 Volts.

Figs. 5A-5B are plots of substrate temperature in °C, measured in still air with a thin thermocouple (Omega, Model CO2-K), as a function of time, in seconds, for a hollow stainless steel cylindrical substrate heated resistively by connection to a capacitor charged to 21 Volts, where Fig. 5A shows the temperature profile over a 4 second time period and Fig. 5B is a detail showing the temperature profile over the first second of heating.

Fig. 6 is a plot comparing purities versus film thickness for flunisolide aerosols generated using a non-treated stainless steel foil substrate (diamonds) and a heat-treated stainless steel foil substrate (squares).

Fig. 7 is a bar graph showing flunisolide vapor purities on non-treated and heat treated steel foils of 304, T-430 and zirconium oxide coated 304. Note that clean steel foil T-430 provides very good purities without heat treatment. Also, zirconium oxide coated steel foils (chemical treatment) provide very good vapor purities compared to both heat treated and non-treated steel foils.

Fig. 8 is a plot comparing purities versus film thickness for eletriptan aerosols generated using a non-treated stainless steel foil substrate (filled circles) and a heat-treated stainless steel foil substrate (open circles).

Fig. 9 is a bar graph comparing purities of alprazolam aerosols generated using a non-treated stainless steel foil substrate, a stainless steel foil substrate heat-treated for 1 hour at 350°C, and a stainless steel foil substrate heat-treated for 6 hours at 350°C.

Fig. 10 is a bar graph comparing purities of bumetanide aerosols generated using stainless steel foil substrates heat-treated for 6 hours at 350°C, stainless steel foil substrates treated with nitric acid and non-treated stainless steel foil substrates.

Fig. 11 is a graph showing budesonide purities on non-treated and heat treated steel foils 304 and T-430 and zirconium oxide coated steel foil 304. Note that clean steel foil T-430 provides very good purities without heat treatment. Also, zirconium oxide coated steel foils (chemical treatment) provide very good vapor purities compared to both heat treated and non-treated steel foils.

**DETAILED DESCRIPTION**

A drug-supply assembly and method for producing a condensation aerosol are disclosed. The drug-supply assembly includes a heat-conductive substrate with an oxidatively inert exterior surface. The drug-supply assembly can be combined with an aerosol device. A film comprising a drug or compound is layered on the exterior surface. The thickness of the film and the exterior surface of the substrate is such that the aerosol formed by vaporizing and condensing the film provides an aerosol containing 10% by weight or less drug-degradation products and at least 50% of the total amount of the drug composition in the film. The methods for treating the exterior surface include heat and chemical treatment and formation of a protective overcoat on a substrate are also provided.

A drug includes any substance that is used in the prevention, diagnosis, alleviation, treatment or cure of a condition. The drug is preferably in a form suitable for thermal vapor delivery, such as an ester, free acid, or free base form. The drugs are typically not recreational drugs. More specifically, the drugs are typically not recreational drugs used for non-medicinal recreational purposes, *e.g.*, habitual use to solely alter one's mood, affect state of consciousness, or to affect a body function unnecessarily, for recreational purposes. The terms "drug", "compound", and "medication" are used interchangeably herein.

The drugs of use in the invention typically have a molecular weight in the range of about 150-700, typically in the range of about 200-650, more typically in the range of 250-600, still more typically in the range of about 250-500, and most typically in the range of about 300-450.

Specific drugs that can be used include, but are not limited to, drugs of one of the following classes: anesthetics, anticonvulsants, antidepressants, antidiabetic agents, antidotes, antiemetics, antihistamines, anti-infective agents, antineoplastics, antiparkisonian drugs, antirheumatic agents, antipsychotics, anxiolytics, appetite stimulants and suppressants, blood modifiers, cardiovascular agents, central nervous system stimulants, drugs for Alzheimer's disease management, drugs for cystic fibrosis management, diagnostics, dietary supplements, drugs for erectile dysfunction, gastrointestinal agents, hormones, drugs for the treatment of alcoholism, drugs for the treatment of addiction, immunosuppressives, mast cell stabilizers, migraine preparations, motion sickness products, drugs for multiple sclerosis management, muscle relaxants, nonsteroidal anti-inflammatories, opioids, other analgesics and stimulants, opthalmic preparations, osteoporosis preparations, prostaglandins, respiratory agents, sedatives and hypnotics, skin and mucous membrane agents, smoking cessation aids, Tourette's syndrome agents, urinary tract agents, and vertigo agents.

Typically, where the drug is an anesthetic, it is selected from one of the following compounds: ketamine and lidocaine.

Typically, where the drug is an anticonvulsant, it is selected from one of the following classes: GABA analogs, tiagabine, vigabatrin; barbiturates such as pentobarbital; benzodiazepines such as clonazepam; hydantoins such as phenytoin; phenyltriazines such as lamotrigine; miscellaneous anticonvulsants such as carbamazepine, topiramate, valproic acid, and zonisamide.

Typically, where the drug is an antidepressant, it is selected from one of the following compounds: amitriptyline, amoxapine, benmoxine, butriptyline, clomipramine, desipramine, dosulepin, doxepin, imipramine, kitanserin, lofepramine, medifoxamine, mianserin, maprotoline, mirtazapine, nortriptyline, protriptyline, trimipramine, venlafaxine, viloxazine, citalopram, cotinine, duloxetine, fluoxetine, fluvoxamine, milnacipran, nisoxetine, paroxetine, reboxetine, sertraline, tianeptine, acetaphenazine, binedaline, brofaromine, cericlamine, clovoxamine, iproniazid, isocarboxazid, moclobemide, phenyhydrazine, phenelzine, selegiline, sibutramine, tranylcypromine, ademetionine, adrafinil, amesergide, amisulpride, amperozide, benactyzine, bupropion, caroxazone, gepirone, idazoxan, metralindole, milnacipran, minaprine, nefazodone, nomifensine, ritanserin, roxindole, S-adenosylmethionine, tofenacin, trazodone, tryptophan, and zalospirone.

Typically, where the drug is an antidiabetic agent, it is selected from one of the following compounds: pioglitazone, rosiglitazone, and troglitazone.

Typically, where the drug is an antidote, it is selected from one of the following compounds: edrophonium chloride, flumazenil, deferoxamine, nalmefene, naloxone, and naltrexone.

Typically, where the drug is an antiemetic, it is selected from one of the following compounds: alizapride, azasetron, benzquinamide, bromopride, buclizine, chlorpromazine, cinnarizine, clebopride, cyclizine, diphenhydramine, diphenidol, dolasetron, droperidol, granisetron, hyoscine, lorazepam, dronabinol, metoclopramide, metopimazine, ondansetron, perphenazine, promethazine, prochlorperazine, scopolamine, triethylperazine, trifluoperazine, triflupromazine, trimethobenzamide, tropisetron, domperidone, and palonosetron.

Typically, where the drug is an antihistamine, it is selected from one of the following compounds: astemizole, azatadine, brompheniramine, carbinoxamine, cetrizine, chlorpheniramine, cinnarizine, clemastine, cyproheptadine, dexmedetomidine, diphenhydramine, doxylamine, fexofenadine, hydroxyzine, loratidine, promethazine, pyrilamine and terfenidine.

Typically, where the drug is an anti-infective agent, it is selected from one of the following classes: antivirals such as efavirenz; AIDS adjunct agents such as dapsone; aminoglycosides such as tobramycin; antifungals such as fluconazole; antimalarial agents such as quinine; antituberculosis agents such as ethambutol; β-lactams such as cefmetazole, cefazolin, cephalexin, cefoperazone, cefoxitin, cephacetrile, cephaloglycin, cephaloridine; cephalosporins, such as cephalosporin C, cephalothin; cephamycins such as cephamycin A, cephamycin B, and cephamycin C, cephapirin, cephradine; leprostatics such as clofazimine; penicillins such as ampicillin, amoxicillin, betacillin, carfecillin, carindacillin, carbenicillin, amylpenicillin, azidocillin, benzylpenicillin, clometocillin, cloxacillin, cyclacillin, methicillin, nafcillin, 2-pentenylpenicillin, penicillin N, penicillin O, penicillin S, penicillin V, dicloxacillin; diphenicillin; heptylpenicillin; and metampicillin; quinolones such as ciprofloxacin, clinafloxacin, difloxacin, grepafloxacin, norfloxacin, ofloxacine, temafloxacin; tetracyclines such as doxycycline and oxytetracycline; miscellaneous anti-infectives such as linezolide, trimethoprim and sulfamethoxazole.

Typically, where the drug is an anti-neoplastic agent, it is selected from one of the following compounds: droloxifene, tamoxifen, and toremifene.

Typically, where the drug is an antiparkisonian drug, it is selected from one of the following compounds: amantadine, baclofen, biperiden, benztropine, orphenadrine, procyclidine, trihexyphenidyl, levodopa, carbidopa, andropinirole, apomorphine, benserazide, bromocriptine, budipine, cabergoline, eliprodil, eptastigmine, ergoline, galanthamine, lazabemide, lisuride, mazindol, memantine, mofegiline, pergolide, piribedil, pramipexole, propentofylline, rasagiline, remacemide, ropinerole, selegiline, spheramine, terguride, entacapone, and tolcapone.

Typically, where the drug is an antirheumatic agent, it is selected from one of the following compounds: diclofenac, hydroxychloroquine and methotrexate.

Typically, where the drug is an antipsychotic, it is selected from one of the following compounds: acetophenazine, alizapride, amisulpride, amoxapine, amperozide, aripiprazole, benperidol, benzquinamide, bromperidol, buramate, butaclamol, butaperazine, carphenazine, carpipramine, chlorpromazine, chlorprothixene, clocapramine, clomacran, clopenthixol, clospirazine, clothiapine, clozapine, cyamemazine, droperidol, flupenthixol, fluphenazine, fluspirilene, haloperidol, loxapine, melperone, mesoridazine, metofenazate, molindrone, olanzapine, penfluridol, pericyazine, perphenazine, pimozide, pipamerone, piperacetazine, pipotiazine, prochlorperazine, promazine, quetiapine, remoxipride, risperidone, sertindole, spiperone, sulpiride, thioridazine, thiothixene, trifluperidol, triflupromazine, trifluoperazine, ziprasidone, zotepine, and zuclopenthixol.

Typically, where the drug is an anxiolytic, it is selected from one of the following compounds: alprazolam, bromazepam, oxazepam, buspirone, hydroxyzine, mecloqualone, medetomidine, metomidate, adinazolam, chlordiazepoxide, clobenzepam, flurazepam, lorazepam, loprazolam, midazolam, alpidem, alseroxlon, amphenidone, azacyclonol, bromisovalum, captodiamine, capuride, carbcloral, carbromal, chloral betaine, enciprazine, flesinoxan, ipsapiraone, lesopitron, loxapine, methaqualone, methprylon, propanolol, tandospirone, trazadone, zopiclone, and zolpidem.

Typically, where the drug is an appetite stimulant, it is dronabinol.

Typically, where the drug is an appetite suppressant, it is selected from one of the following compounds: fenfluramine, phentermine and sibutramine.

Typically, where the drug is a blood modifier, it is selected from one of the following compounds: cilostazol and dipyridamol.

Typically, where the drug is a cardiovascular agent, it is selected from one of the following compounds: benazepril, captopril, enalapril, quinapril, ramipril, doxazosin, prazosin, clonidine, labetolol, candesartan, irbesartan, losartan, telmisartan, valsartan, disopyramide, flecanide, mexiletine, procainamide, propafenone, quinidine, tocainide, amiodarone, dofetilide, ibutilide, adenosine, gemfibrozil, lovastatin, acebutalol, atenolol, bisoprolol, esmolol, metoprolol, nadolol, pindolol, propranolol, sotalol, diltiazem, nifedipine, verapamil, spironolactone, bumetanide, ethacrynic acid, furosemide, torsemide, amiloride, triamterene, and metolazone.

Typically, where the drug is a central nervous system stimulant, it is selected from one of the following compounds: amphetamine, brucine, caffeine, dexfenfluramine, dextroamphetamine, ephedrine, fenfluramine, mazindol, methyphenidate, pemoline, phentermine, sibutramine, and modafinil.

Typically, where the drug is a drug for Alzheimer's disease management, it is selected from one of the following compounds: donepezil, galanthamine and tacrin.

Typically, where the drug is a drug for cystic fibrosis management, it is selected from one of the following compounds: tobramycin and cefadroxil.

Typically, where the drug is a diagnostic agent, it is selected from one of the following compounds: adenosine and aminohippuric acid.

Typically, where the drug is a dietary supplement, it is selected from one of the following compounds: melatonin and vitamin-E.

Typically, where the drug is a drug for erectile dysfunction, it is selected from one of the following compounds: tadalafil, sildenafil, vardenafil, apomorphine, apomorphine diacetate, phentolamine, and yohimbine.

Typically, where the drug is a gastrointestinal agent, it is selected from one of the following compounds: loperamide, atropine, hyoscyamine, famotidine, lansoprazole, omeprazole, and rebeprazole.

Typically, where the drug is a hormone, it is selected from one of the following compounds: testosterone, estradiol, and cortisone.

Typically, where the drug is a drug for the treatment of alcoholism, it is selected from one of the following compounds: naloxone, naltrexone, and disulfiram.

Typically, where the drug is a drug for the treatment of addiction it is buprenorphine.

Typically, where the drug is an immunosupressive, it is selected from one of the following compounds: mycophenolic acid, cyclosporin, azathioprine, tacrolimus, and rapamycin.

Typically, where the drug is a mast cell stabilizer, it is selected from one of the following compounds: cromolyn, pemirolast, and nedocromil.

Typically, where the drug is a drug for migraine headache, it is selected from one of the following compounds: almotriptan, alperopride, codeine, dihydroergotamine, ergotamine, eletriptan, frovatriptan, isometheptene, lidocaine, lisuride, metoclopramide, naratriptan, oxycodone, propoxyphene, rizatriptan, sumatriptan, tolfenamic acid, zolmitriptan, amitriptyline, atenolol, clonidine, cyproheptadine, diltiazem, doxepin, fluoxetine, lisinopril, methysergide, metoprolol, nadolol, nortriptyline, paroxetine, pizotifen, pizotyline, propanolol, protriptyline, sertraline, timolol, and verapamil.

Typically, where the drug is a motion sickness product, it is selected from one of the following compounds: diphenhydramine, promethazine, and scopolamine.

Typically, where the drug is a drug for multiple sclerosis management, it is selected from one of the following compounds: bencyclane, methylprednisolone, mitoxantrone, and prednisolone.

Typically, where the drug is a muscle relaxant, it is selected from one of the following compounds: baclofen, chlorzoxazone, cyclobenzaprine, methocarbamol, orphenadrine, quinine, and tizanidine.

Typically, where the drug is a nonsteroidal anti-inflammatory, it is selected from one of the following compounds: aceclofenac, acetaminophen, alminoprofen, amfenac, aminopropylon, amixetrine, aspirin, benoxaprofen, bromfenac, bufexamac, carprofen, celecoxib, choline, salicylate, cinchophen, cinmetacin, clopriac, clometacin, diclofenac, diflunisal, etodolac, fenoprofen, flurbiprofen, ibuprofen, indomethacin, indoprofen, ketoprofen, ketorolac, mazipredone, meclofenamate, nabumetone, naproxen, parecoxib, piroxicam, pirprofen, rofecoxib, sulindac, tolfenamate, tolmetin, and valdecoxib.

Typically, where the drug is an opioid, it is selected from one of the following compounds: alfentanil, allylprodine, alphaprodine, anileridine, benzylmorphine, bezitramide, buprenorphine, butorphanol, carbiphene, cipramadol, clonitazene, codeine, dextromoramide, dextropropoxyphene, diamorphine, dihydrocodeine, diphenoxylate, dipipanone, fentanyl, hydromorphone, L-alpha acetyl methadol, lofentanil, levorphanol, meperidine, methadone, meptazinol, metopon, morphine, nalbuphine, nalorphine, oxycodone, papaveretum, pethidine, pentazocine, phenazocine, remifentanil, sufentanil, and tramadol.

Typically, where the drug is an other analgesic it is selected from one of the following compounds: apazone, benzpiperylon, benzydramine, caffeine, clonixin, ethoheptazine, flupirtine, nefopam, orphenadrine, propacetamol, and propoxyphene.

Typically, where the drug is an opthalmic preparation, it is selected from one of the following compounds: ketotifen and betaxolol.

Typically, where the drug is an osteoporosis preparation, it is selected from one of the following compounds: alendronate, estradiol, estropitate, risedronate and raloxifene.

Typically, where the drug is a prostaglandin, it is selected from one of the following compounds: epoprostanol, dinoprostone, misoprostol, and alprostadil.

Typically, where the drug is a respiratory agent, it is selected from one of the following compounds: albuterol, ephedrine, epinephrine, fomoterol, metaproterenol, terbutaline, budesonide, ciclesonide, dexamethasone, flunisolide, fluticasone propionate, triamcinolone acetonide, ipratropium bromide, pseudoephedrine, theophylline, montelukast, and zafirlukast.

Typically, where the drug is a sedative and hypnotic, it is selected from one of the following compounds: butalbital, chlordiazepoxide, diazepam, estazolam, flunitrazepam, flurazepam, lorazepam, midazolam, temazepam, triazolam, zaleplon, zolpidem, and zopiclone.

Typically, where the drug is a skin and mucous membrane agent, it is selected from one of the following compounds: isotretinoin, bergapten and methoxsalen.

Typically, where the drug is a smoking cessation aid, it is selected from one of the following compounds: nicotine and varenicline.

Typically, where the drug is a Tourette's syndrome agent, it is pimozide.

Typically, where the drug is a urinary tract agent, it is selected from one of the following compounds: tolteridine, darifenicin, propantheline bromide, and oxybutynin.

Typically, where the drug is a vertigo agent, it is selected from one of the following compounds: betahistine and meclizine.

As used herein the term "a" and "the" in combination with a particular reference means single and plural unless the context clearly indicates otherwise. For example, "a drug" includes a single drug species or a combination of drug species.

The terms "drug composition" as used herein refers to a composition that comprises only pure drug, two or more drugs in combination, or one or more drugs in combination with additional components. Additional components can include, for example, pharmaceutically acceptable excipients, carriers, and surfactants.

The term "drug degradation product" as used herein refers to a compound resulting from a chemical modification of the drug compound during a drug vaporization-condensation process. The modification, for example, can be the result of a thermally or photochemically or catalytically induced reaction. Such reactions include, without limitation, oxidation and hydrolysis.

The term "fraction drug degradation product" as used herein refers to the quantity of drug degradation products present in the aerosol particles divided by the quantity of drug plus drug degradation product present in the aerosol, i.e. (sum of quantities of all drug degradation products present in the aerosol)/((quantity of drug composition present in the aerosol) + (sum of quantities of all drug degradation products present in the aerosol)). The term "percent drug degradation product" as used herein refers to the fraction drug degradation product multiplied by 100%, whereas "purity" of the aerosol refers to 100% minus the percent drug degradation products.

The term "effective therapeutic dose" means the amount required to achieve the desired effect or efficacy, *e.g.*, abatement of symptoms or cessation of the episode, in a subject (*e.g.*, a mammal, such as a human). The dose of a drug delivered in the thermal vapor refers to a unit dose amount that is generated by heating of the drug under defined delivery conditions. A "unit dose amount" is the total amount of drug in a given volume of inhaled thermal vapor.

The term "exterior surface" refers to the exterior-most boundary of a substrate. Typically, the exterior surface consists of the exterior-most 2 nm, 20 nm, or 200 nm of a substrate. In another aspect, the "exterior surface" is a surface in direct contact with a drug or compound comprising a layer disposed upon a heat conductive substrate. The layer can be a discreet layer upon the substrate, such that the exterior surface layer composition may be different from the bulk material of the substrate (*e.g.*, an overcoat material). Suitable exterior surfaces are typically oxidatively inert. Examples of suitable substrates include: steel, stainless steel, aluminum, chromium, copper, iron, titanium, conducting ceramics, and alloys of thermally conducting metals. Examples of suitable exterior substrates include: metal oxides ($MO$, $M_2O_3$, $MO_2$), where the oxidation state of M is +2 (*e.g.*, Fe, Ca, Sr, Zn) or +3 (*e.g.*, Fe, Al, Cr, Mo, Lanthanides) or +4 (*e.g.*, Zr, Ce, Si and lanthanides); mixed metal oxides ($M_1M_2Ox$, where $M_1$ and $M_2$ are metals having oxidation states of +2, +3, or +4; *e.g.*, $MgOAl_2O_2$, $FeOFe_2O_c$, $ZnOAl_2O_3$).

The term "substrate interior" refers to the portion of a solid or bulk material of the substrate, excluding the exterior surface.

The term "metal oxide-enriched exterior surface" refers to the exterior surface of a substrate, which contains a greater amount of one or more metal oxides than does a reference exterior surface of such a substrate material. Compared to the oxide layer of the reference exterior surface, the metal oxide-enriched exterior surface may be distinguished by a greater depth of the oxide layer, an increased content of a specific metal oxide species in the exterior surface, and/or a reduced content of a specific non-oxidized metal species in the exterior surface. Typically, when the substrate comprises stainless steel, the reference exterior surface comprises predominantly iron oxide and chromium oxide layer of less than 5 nm in thickness. In contrast, a metal oxide-enriched exterior surface of a stainless steel substrate may comprise an iron oxide and chromium oxide layer of 7.5 nm or more in thickness, or may contain a greater amount of oxidation resistant metal oxides in the exterior-most 5 nm than does the reference exterior surface, or may contain a lesser amount of metals/alloys such as iron, nickel, chromium, molybdenum, silicon, aluminum or manganese in the exterior-most 5 nm than does the reference exterior surface. Methods of analyzing the exterior surface of a metal substrate are known in the art. For example, static or dynamic secondary ion mass spectroscopy (SIMS), x-ray photoelectron spectroscopy/electron spectroscopy for chemical analysis (XPS/ESCA), ellipsometry, and Auger electron spectroscopy (AES) can be used to analyze the exterior surface.

The term "reference exterior surface" of a substrate material refers to the exterior surface layer that forms upon brief (*e.g.* 1 month) exposure of the material to air at room temperature. To form a reference exterior surface, one means is to process bulk metal substrate material, *e.g.*, by slicing the bulk material, such that a portion of the interior of the bulk material becomes the exterior surface of the processed material. Storage of the processed material in air at room temperature for a brief period (*e.g.*, I month, with storage for as little as I day generally giving a similar outcome) results in formation of a reference exterior surface.

"Thermal vapor" refers to a vapor phase, aerosol, or mixture of aerosol-vapor phases, formed by, for example, heating. The thermal vapor may comprise a drug and optionally a carrier, and may be formed by heating the drug and optionally a carrier. Vapor phase refers to a gaseous phase. An aerosol phase comprises solid and/or liquid particles suspended in a gaseous phase.

A "treated exterior surface" refers to an exterior surface that has been subjected to heat, and/or chemical treatment/overcoat modifications. A treated surface may result in metal oxide-enrichment of the surface. An overcoat provides an oxidation resistant/protective coat of chemically less reactive metals/metal oxides to a substrate.

In one aspect, the invention provides a drug-supply assembly for use in an aerosol device, for producing an aerosol of a compound such as a drug compound. The aerosol is produced by a vaporization-condensation technique. The assembly is particularly suited for use in a device for inhalation therapy for delivery of a compound (*e.g.*, a therapeutic agent) to the lungs of a subject, for local or systemic treatment. The assembly is also suited for use in a device that generates an air stream, for application of drug-aerosol particles to a target site. For example, a stream of air carrying drug-aerosol particles can be applied to treat an acute or chronic skin condition, can be applied during surgery at the incision site, or can be applied to an open wound. The assembly and its use in an inhalation device are described.

Among the advantages and features of a drug-supply assembly of the invention is the formation of substantially pure aerosol particles upon vaporization of a drug from the assembly by application of heat to the drug.

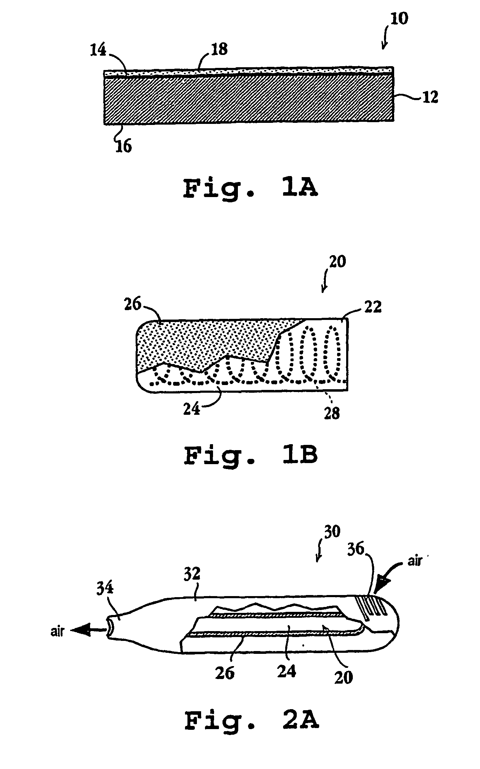

A drug-supply assembly for use in a condensation aerosol device according to one embodiment of the invention is shown in cross-sectional view in Fig. 1A. Drug-supply assembly **10** is comprised of a heat-conductive substrate **12**. In one aspect, the substrate comprises a metal and/or metal al loy. Examples of metals appropriate for the substrate include aluminum, titanium, iron, copper, stainless steel, and the like. Heat-conductive substrate **12** has an exterior surface **14** and a substrate interior **16**. The exterior surface **14** can be an overlay of an oxidatively inert material applied to the heat conductive substrate. In another aspect, the exterior surface **14** comprises an oxide layer of the substrate **12** obtained by heat or chemical treatment of the substrate **12**. The substrate can be of virtually any geometry; the square or rectangular configuration shown in Fig. 1A merely exemplary.

Where an overlay of an oxidatively inert material is to be performed, the substrate is typically a metal, ceramic, glass or other material. An overlay layer can be applied to a substrate by any number of methods known in the art. For example, an overlay layer can be applied to a substrate using solution casting or suspension casting techniques. In general, solution cast routes are advantageous because they provide homogeneous structures and ease of processing. With solution cast routes, the overlay layer may be easily fabricated by spin, spray or dip coating. Suspension casting still provides the possibility of spin, spray or dip coating but more heterogeneous structures than with solution casting are expected. For systems where one or more materials are soluble in a common solvent, the film can be fabricated by solution casting. This allows for soluble materials to be dissolved and a composite film formed in a single step upon solvent evaporation. In suspension casting, one or more materials are suspended and/or dissolved in a common solvent. Suspension casting is a rather general technique applicable to a wide range of chemical species. In one application of suspension casting, the material is dissolved in an appropriate solvent, a second material is then suspended in this solution and the resulting mixture is used to dip coat or spray coat a substrate.

With continuing reference to Fig. 1A, deposited on all or a portion of the exterior surface **14** of the substrate **12** is a film **18** comprising a drug. Thus, the exterior surface **14** is in contact with molecules of a drug to be aerosolized. As discussed above, the exterior surface **14** is oxidatively inert and/or is selected or modified to avoid oxidative or chemical degradation of the film **18** (*e.g.*, before and/or during heating of the substrate **12** and exterior surface **14**). In one embodiment of the invention, the exterior surface **14** is treated to improve the oxide content of the exterior surface **14.**

In addition to the substrate having an exterior surface, the substrate should have an exterior surface with relatively few or substantially no surface irregularities, so that a molecule of a compound vaporized from a film on the exterior surface is unlikely to acquire sufficient energy through contact with (i) other hot vapor molecules, (ii) hot gases surrounding the area, or (iii) the substrate surface to result in cleavage of chemical bonds and hence compound decomposition. To minimize the energy input to a vaporized compound that might result in chemical decomposition, the vaporized compound should transition rapidly from the heated surface or surrounding heated gas to a cooler environment. While a vaporized compound from a surface may transition through Brownian motion or diffusion, the temporal duration of this transition may be impacted by the extent of the region of elevated temperature at the surface which is established by the velocity gradient of gases over the surface and the physical shape of surface. A high velocity gradient (a rapid increase in velocity gradient near the surface) results in minimization of the hot gas region above the heated surface and decreases the time of transition of the vaporized compound to a cooler environment. Likewise, a smoother surface facilitates this transition, as the hot gases and compound vapor are not precluded from rapid transition by being trapped in, for example, depressions, pockets or pores on the substrate or exterior surface. For the reasons stated above, non-preferred substrates are those that have a substrate density of less than 0.5 g/cc.

With continuing reference to Fig. 1A, film **18** comprising a drug has a thickness of between about 0.05 $\mu$m and 20 $\mu$m. Film deposition is achieved by a variety of methods, depending in part on the physical properties of the drug and on the desired drug film thickness. Exemplary methods include, but are not limited to, preparing a solution of drug in a solvent, applying the solution to the exterior surface and removing the solvent to leave a film of drug. The drug solution can be applied by dipping the substrate into the solution, spraying, brushing or otherwise applying the solution to the substrate. For example, a film comprising a drug can be applied to a substrate using solution casting and/or suspension casting techniques. In general, solution cast routes are advantageous because they provide homogeneous structures and ease of processing. With solution cast routes, the film may be easily fabricated by spin, spray or dip coating. Suspension casting still provides the possibility of spin, spray or dip coating but more heterogeneous structures than with solution casting are expected. For systems where one or more drugs and/or carriers or other materials are soluble in a common solvent, the film can be fabricated by solution casting. This allows for soluble drugs to be dissolved and a composite film formed in a single step upon solvent evaporation. In suspension casting, one or more drugs, carriers, or other materials are suspended and/or dissolved in a common solvent. Suspension casting is a rather general technique applicable to a wide range of chemical species. In one application of suspension casting, the drug is dissolved in an appropriate solvent, a second compound or drug is then suspended in this solution and the resulting mixture is used to dip coat or spray coat an exterior surface. Alternatively, a melt of the drug can be prepared and applied to the substrate. For drugs that are liquids at room temperature, thickening agents can be admixed with the drug to permit application of a solid drug film.

In another aspect, the drug film is treated to improve the thermal stability of the flow, *e.g.*, melt viscosity and fluidity, uniformity of heating, and the like, at elevated temperatures compared to untreated drug. Such increased thermal stability may also increase the shelf-life stability of the drug in the film by decreasing the shelf-life degradation of the drug composition. Alternatively, or in addition, the increased thermal stability of the drug in the film may serve to increase the purity of the condensation aerosol formed upon heating the drug film, by decreasing the thermal degradation of the drug during heating and subsequent vaporization of the film comprising the drug.

Fig. 1B is a perspective, cut-away view of an alternative geometry of the drug-supply assembly for use in a condensation aerosol device. Drug-supply assembly **20** is comprised of a cylindrically-shaped substrate **22** formed from a heat-conductive material. In the embodiment shown in Fig. 1B, substrate **22** has a metal-oxide enriched or oxidation resistance exterior surface **24**. Deposited on the exterior surface **24** of the substrate **22** is a film **26** comprising a drug. As will be described in more detail below, in use, the substrate **22** of assembly **20** is heated to vaporize all or a portion of the film **26**. Control of air flow across the substrate surface during vaporization produces the desired size of drug-aerosol particles. In Fig. 1B, the film **26** and exterior surface **24** is partially cut-away to expose a heating element **28** in thermal communication with substrate **22.** The heating element **28** may also comprise a sensor to maintain an operating temperature of 300-500 °C. Operation at higher temperatures is also possible. The heater can be in the form of a resistive coil, thick film, or sheet heater. A suitable thermocouple attachment is made to the sensor for accurate temperature monitoring. Other heating elements are suitable including, but not limited to, a solid chemical fuel, chemical components that undergo an exothermic reaction, inductive heat, and the like. Heating of the substrate by conductive heating is also suitable. One exemplary heating source is described in International Patent Application entitled, "SELF-CONTAINED HEATING UNIT AND DRUG-SUPPLY UNIT EMPLOYING SAME," filed May 20, 2004, published as WO 2004/104490. For example, the substrate can be hollow with a heating element inserted into the hollow space or solid with a heating element incorporated into the substrate. The heating element **28** in the embodiment shown in Fig. 1B takes the form of an electrical resistive wire that produces heat when a current flows through the wire.

Fig. 2A is a perspective view of a drug-delivery device that incorporates a drug-supply assembly for use in a condensation aerosol device similar to that shown in Fig. 1B. Device **30** includes a housing **32** with a tapered end **34** for insertion into the mouth of a user. On the end opposite tapered end **34,** the housing has one or more openings, such as slot 36, for air intake when a user places the device in the mouth and inhales a breath. Disposed within housing **32** is drug-supply assembly **20,** visible in the cut-away portion of the figure. Drug-supply assembly **20** includes a substrate with an exterior surface **24** coated with a film **26** comprising a drug to be delivered to the user. The assembly **20** can be rapidly heated to a temperature sufficient to vaporize all or a portion of the film **26** comprising the drug to form a drug vapor that becomes entrained in the stream of air during inhalation, thus forming drug-aerosol particles. Heating of the drug-supply assembly **20** is accomplished by, for example, an electrically-resistive wire embedded or inserted into the substrate and connected to a battery disposed in the housing. Substrate heating can be actuated by a user-activated button on the housing or via breath actuation.

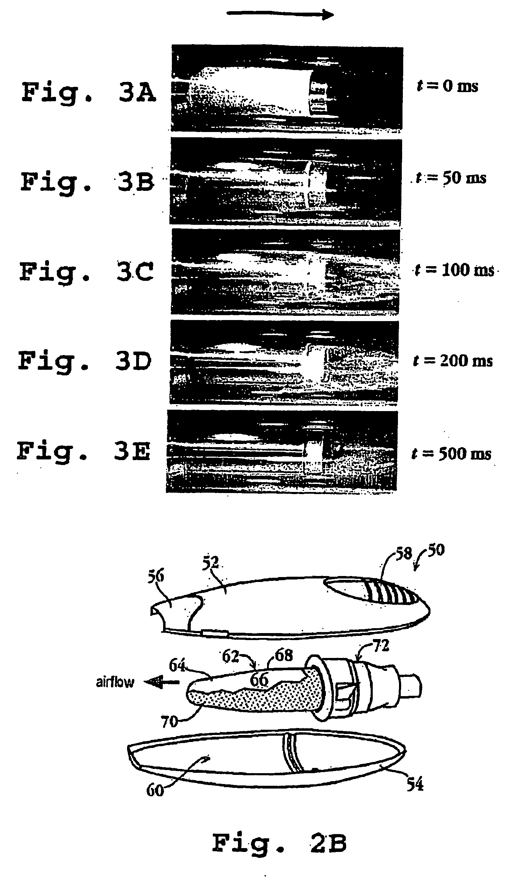

Fig. 2B shows another drug-delivery device that incorporates a drug-supply assembly, where the device components are shown in unassembled form. Inhalation device **50** is comprised of an upper external housing member **52** and a lower external housing member **54** that fit together. The downstream end of each housing member is gently tapered for insertion into a user's mouth, best seen on upper housing member **52** at downstream end **56**. The upstream end of the upper **52** and lower **54** housing members are slotted, as seen best in the figure in the upper housing member at **58,** to provide for air intake when a user inhales. The upper **52** and lower **54** housing members when fitted together define a chamber **60**. Positioned within chamber **60** is a drug-supply assembly **62,** shown in a partial cut-away view. The drug supply unit has a tapered, substantially cylindrical substrate **64** coated with a film **66** comprising a drug on the exterior surface **68** of substrate **64**. Visible in the cut-away portion of the drug-supply unit is an interior region **70** of the substrate containing a heat element or heating substance suitable to generate heat. The heat element or heating substance can be a solid chemical fuel, chemical reagents that mix exothermically, electrically resistive wire and the like. A power supply source, if needed for heating, and any necessary valving for the inhalation device are contained in end piece **72** which is in electrical, mechanical and/or thermal communication with substrate **64.**

In a typical embodiment, the device includes a gas-flow control valve disposed upstream of the drug-supply assembly for limiting gas-flow rate through the condensation region. For example, the gas flow control valve limits air flow through the chamber as air is drawn by the user's mouth into and through the chamber. In a specific embodiment, the gas-flow valve includes an inlet port communicating with the chamber, and a deformable flap adapted to divert or restrict air flow away from the inlet port increasingly, with increasing pressure drop across the valve. In another embodiment, the gas-flow valve includes an actuation switch coupled with valve movement such that in response to an air pressure differential across the valve, the valve acts to close the switch. In still another embodiment, the gas-flow valve includes an orifice designed to limit airflow rate into the chamber.

The device may also include a bypass valve communicating with the chamber downstream of the unit for offsetting the decrease in airflow produced by the gas-flow control valve, as the user draws air into the chamber. The bypass valve cooperates with the gas-control valve to control the flow through the condensation region of the chamber as well as the total amount or volume of air being drawn through the device. Thus, the total volumetric airflow through the device is the sum of the volumetric airflow rate through the gas-control valve and the volumetric airflow rate through the bypass valve. The gas control valve acts to limit air drawn into the device to a preselected level, *e.g.*, 15 L/minute, corresponding to the selected air-flow rate for producing aerosol particles of a selected size. Once this selected airflow level is reached, additional air drawn into the device creates a pressure drop across the bypass valve which then accommodates airflow through the bypass valve into the downstream end of the device adjacent the user's mouth. Thus, the user senses a full breath being drawn in, with the two valves distributing the total airflow between desired airflow rate and bypass airflow rate.

These valves may be used to control the gas velocity through the condensation region of the chamber and hence to control the particle size of the aerosol particles produced by vapor condensation. More rapid airflow dilutes the vapor such that it condenses into smaller particles. In other words, the particle size distribution of the aerosol is determined by the concentration of the compound vapor during condensation. This vapor concentration is, in turn, determined by the extent to which airflow over the surface of the heating substrate dilutes the evolved vapor. Thus, to achieve smaller or larger particles, the gas velocity through the condensation region of the chamber may be altered by modifying the gas-flow control valve to increase or decrease the volumetric airflow rate. For example, to produce condensation particles having a mass median aerodynamic diameter (MMAD) in the size range 1-3.5 $\mu$m, the chamber may have substantially smooth-surfaced walls, and the selected gas-flow rate may be in the range of 4-50 L/minute.

Additionally, as will be appreciated by one of skill in the art, particle size may be also altered by modifying the cross-section of the chamber condensation region to increase or decrease linear gas velocity for a given volumetric flow rate, and/or the presence or absence of structures that produce turbulence within the chamber. Thus, for example, to produce condensation particles in the size range 20-100 nm MMAD, the chamber may provide gas-flow barriers for creating air turbulence within the condensation chamber. These barriers are typically placed within a few thousands of an inch from the substrate surface.

The heat source in one embodiment is effective to supply heat to the substrate at a rate that achieves a substrate temperature of at least 150°C, at least 250 °C, at least 350 °C, or at least 400 °C, and produces substantially complete volatilization of the drug composition from the substrate within a period of 2 seconds, within a period of 1 second, or more typically within a period of 0.5 seconds. Suitable heat sources include resistive heating devices that are supplied with electrical current at a rate sufficient to achieve rapid heating to a substrate temperature of at least 150°C, 200°C, 250 °C, 300 °C, or 350 °C within 50-500 ms, but typically in the range of 50-200 ms. Heat sources or devices that contain a chemically reactive material which undergoes an exothermic reaction upon actuation, *e.g.*, by a spark or heat element, such as flashbulb type heaters and the heating source described in the above-cited International Application entitled, "SELF-CONTAINED HEATING UNIT AND DRUG-SUPPLY UNIT EMPLOYING SAME," WO2004/104490, also suitable. In particular, heat sources that generate heat by exothermic reaction, where the chemical "load" of the source is consumed in a period of between 50-500 msec or less, are generally suitable, with good thermal coupling between the heat source and substrate.

Figs. 3A-3E are high speed photographs showing the generation of aerosol particles from a drug-supply assembly. Fig. 3A shows a cylindrical stainless steel substrate about 2 cm in length coated with a film comprising a drug. Prior to coating the substrate with the film, the steel substrate was heated about three times in air to a temperature of approximately 400°C for a period of approximately 2 seconds to form a metal-oxide enriched exterior surface. The drug-coated substrate was placed in a chamber through which a stream of air was flowing in an upstream-to-downstream direction (indicated by the arrow in Fig. 3A) at rate of about 15 L/min. The substrate was electrically heated and the progression of drug vaporization monitored by real-time photography. Figs. 3B-3E show the sequence of drug vaporization and aerosol generation at time intervals of 50 milliseconds (msec), 100 msec, 200 msec, and 500 msec, respectively. The white cloud of drug-aerosol particles formed from the drug vapor entrained in the flowing air is visible in the photographs. Complete vaporization of the drug film was achieved by 500 msec.

The drug-supply assembly generates a drug vapor that can readily be mixed with gas to produce an aerosol for inhalation or for delivery, to a topical site, typically by spray nozzle, for a variety of treatment regimens, including acute or chronic treatment of a skin condition, or administration of a drug to an incision site during surgery or to an open wound. Rapid vaporization of the drug film occurs with minimal thermal decomposition of the drug when the substrate advantageously has a treated exterior surface.

As discussed above, the drug-supply assembly for use in a condensation aerosol device includes a film comprising a drug formed on a substrate having an exterior surface. In one aspect, the surface of the substrate is treated to provide a treated exterior surface. In one embodiment, the film comprises two or more drugs. In another embodiment, the film comprises a pure drug. A film, in one embodiment of the invention, has a thickness of between about 0.05-20 $\mu$m, between 0.1-15 $\mu$m, between 0.2-10 $\mu$m, or 0.5-10 $\mu$m, but is most typically 1-10 $\mu$m. The film thickness for a given drug or drug composition is such that drug-aerosol particles formed by vaporizing the drug or drug composition by heating the substrate and entraining the vapor in a gas stream have (i) 10% by weight or less drug-degradation product, typically 5% by weight or less, and commonly 2.5% by weight or less, and (ii) at least 50% of the total amount of drug composition contained in the film. The area of the treated exterior surface of the substrate on which the drug film is formed is selected to achieve an effective therapeutic dose of the drug aerosol. In some aspects, the substrate is treated by means including heat treatment and chemical treatment to yield an exterior surface with an enhanced oxidation resistance, metal oxide layer thickness, enhanced content of one or more metals or metal oxides in the oxide layer, or a decreased amount of one or more non-oxidized metal species.

The substrate may be treated by a variety of means to enrich or coat the substrate exterior with oxidation resistant materials or metal oxides to generate an exterior surface comprising a metal oxide layer. One means of treating the substrate surface is heat treatment. In one aspect, the invention includes a method of preparing a drug-supply assembly for use in an aerosol device, wherein the assembly comprises a heat-conductive metal substrate comprising heat-treating the metal substrate and coating at least a portion of said substrate with a film comprising a drug. The film comprising a drug is coated on the exterior surface of the metal substrate, where the film thickness and exterior surface are such that an aerosol formed by vaporizing the drug film by heating the substrate and condensing the vaporized drug composition contains 10% by weight or less drug-degradation products and at least 50% of the total amount of the drug composition in the film. The substrate may be heat-treated (prior to coating with the film) at a temperature between approximately 60 °C and 800 °C, more typically between 100 °C and 500 °C, or most commonly between 200 °C and 400 °C. At such temperatures, the substrate may acquire a desirable exterior surface in about 1 second at around 400°C, although longer periods of heat-treatment, *e.g.*, for minutes, hours, or days may result in a desirable exterior surface. However, heat treatment of the substrate may not enhance the purity of drugs, if the substrate is already oxidation resistant or metal oxide enriched prior to heat treatment. In general, when a lower temperature is used for the treatment, longer treatment duration is typically performed, and when a higher temperature is used for the treatment, shorter treatment duration is typically performed. Thus, the substrate may be heat-treated for a period of between 1 second and 5 days, typically between 5 minutes and 24 hours, and commonly between 15 minutes and 8 hours. The treatment may be carried out in air, dried air, oxygen, or partial vacuum with oxygen present, among other conditions. In a specific example, heat treatment includes heating the substrate for at least 6 hours at 350 °C; or at least 2 seconds, and typically at least 5 seconds at 500 °C in air.

When the substrate is a metal, heat treatment generally results in a metal oxide-enriched substrate exterior surface characterized by a thicker layer of metal oxide than that of the reference exterior surface that is not heat treated. Typically, the reference exterior surface has a passive layer with an oxide layer thickness for stainless steel substrates of < 5 nm, whereas the heat-treated substrate has an oxide layer thickness of > 7.5 nm, > 10 nm, and typically > 20 nm. Heat treatment produces a more fully oxidized exterior surface, i.e., an exterior surface containing a reduced quantity of non-oxidized metal species compared with the reference exterior surface. In the case where the metal substrate is steel or stainless steel, the exterior surface upon heat treatment becomes enriched, among other species, with iron oxide and depleted of metallic iron. In the case wherein the metal substrate is aluminum, heat treatment increases the substrate exterior aluminum oxide layer thickness, or increases the aluminum oxide content of the substrate exterior surface, or decreases the content of metallic aluminum or other metals in the exterior surface.

Another method of producing a treated exterior surface of a substrate is chemical treatment or forming a protective overcoat of the substrate surface. Chemical treatment can be done with acids (*e.g.*, hydrofluoric acid, sulfuric acid, nitric acid, hydrochloric acid, formic acid, and citric acid), bases (*e.g.*, sodium hydroxide), water, salt solutions (*e.g.*, chloride or phosphate salts), with and without application of an electric potential across the metal substrate. In the case of nitric acid treatment of a stainless steel substrate, for example, the treated exterior surface is primarily enriched in metal oxide. This is reflected in an increase in chromium oxide content of the substrate's exterior surface with a corresponding decrease in the content of metallic iron and chromium in the exterior-most portion of the substrate in contact with a drug film. Chemical treatment is typically conducted by soaking a metal substrate in a chemical solution for a period of time, such as, for example, 30 minutes, 15 minutes, 5 minutes, or 3 minutes. Additionally, sonication and/or heat may also be used. After chemical treatment, the substrate is typically washed and dried. Chemically less reactive metals or metal oxides or ceramics (*e.g.*, gold platinum, zirconium oxide, silicon carbide) can be deposited (*e.g.*, by vapor deposition, electroplating, dip coating, spray coating, and the like) onto the substrate surface to provide a chemically altered exterior surfaces of metallic substrates. After forming a chemical overcoat, the substrate is typically washed and dried with appropriate solvents.

The above treatment approaches are applicable to a diversity of metals and alloys including, without limitation, steel, stainless steel, aluminum, chromium, copper, iron, titanium, and the like, with aluminum, copper, and steel (including stainless steel), being typically used.

In studies conducted in support of the invention, a variety of drugs were deposited as a film on a heat-conductive, impermeable substrate and the substrate was heated to a temperature sufficient to generate a thermal vapor. Purity of drug-aerosol particles in the thermal vapor was determined. To determine the percent fraction of drug degradation products, the aerosol is typically collected in a trap, such as a filter, glass wool, an impinger, a solvent trap, or a cold trap, with collection in a filter being the common technique used. The trap is then extracted with a solvent, *e.g.* acetonitrile, and the extract subjected to analysis by any of a variety of analytical methods known in the art, with gas and liquid chromatography methods typically being used, and high performance liquid chromatography (HPLC) particularly useful. The gas or liquid chromatography method includes a detector system such as a mass spectrometry detector or ultraviolet absorption detector. Ideally, the detector system allows determination of the quantity of the components of the drug composition and drug degradation product by weight. This is achieved in practice by measuring the signal obtained upon analysis of one or more known mass(es) of components of the drug composition or drug degradation product (standards) and comparing the signal obtained upon analysis of the aerosol to that obtained upon analysis of the standard(s), an approach well known in the art. In many cases, the structure of a drug degradation product may not be known or a standard of the drug degradation product may not be available. In such cases, it is acceptable to calculate the weight fraction of the drug degradation product by assuming that the drug degradation product has an identical response coefficient (*e.g.*, for ultraviolet absorption detection, identical extinction coefficient) to the drug component or components in the drug composition. When conducting such analysis, for purposes of practicality, drug degradation products present at less than a very small fraction of the drug compound, *e.g.*, less than 0.2% or 0.1% or 0.03% of the drug compound, are generally excluded from analysis. Because of the frequent necessity to assume an identical response coefficient between drug and drug degradation product in calculating a weight percentage of drug degradation product, it is preferred to use an analytical approach in which such an assumption has a high probability of validity. In this respect, high performance liquid chromatography with detection by absorption of ultraviolet light at 225 nm is typically used. UV absorption at other than 225 nm, most commonly 250 nm, is used for detection of compounds in cases where the compound absorbs substantially more strongly at 250 nm or for other reasons one skilled in the art would consider detection at 250 nm the most appropriate means of estimating purity by weight using HPLC analysis. In certain cases where analysis of the drug by UV is not viable, other analytical tools such as GC/MS or LC/MS may be used to determine purity.

Exemplary studies in support of the invention (which should not be construed to limit the invention) were carried out on four different substrate materials: 302/304 stainless steel (foil and cylinder), T-430 stainless steel foil, 302/304 steel foil coated with zirconium oxide and a stainless steel cylinder. The substrates were either untreated or treated as described herein.

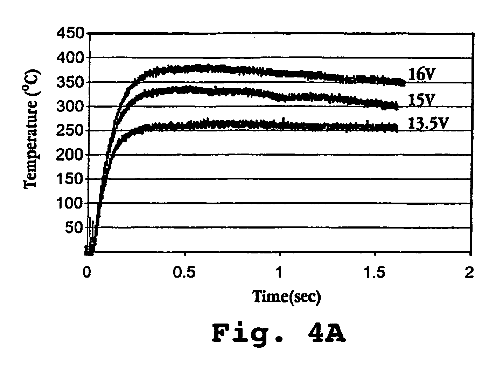

To volatilize a drug film coated on the stainless steel foil substrate, the stainless steel substrate was resistively heated by placing the substrate between a pair of electrodes connected to a capacitor that was charged to between 14-17 Volts. Fig. 4A is a plot of temperature increase in °C, measured in no airflow with a thin thermocouple (Omega, Model CO2-K), against time, in seconds, for a stainless steel foil substrate resistively heated by charging the capacitor to 13.5 V (lower line), 15 V (middle line), and 16 V (upper line). When charged with 13.5 V, the substrate temperature increase was about 250 °C within about 200-300 milliseconds. As the capacitor voltage increased, the peak temperature of the substrate also increased. Charging the capacitor to 16V heated the foil substrate temperature about 375 °C in 200-300 milliseconds (to a maximum temperature of about 400 °C). Surface substrate temperature was found to be similar for both untreated and treated substrates.

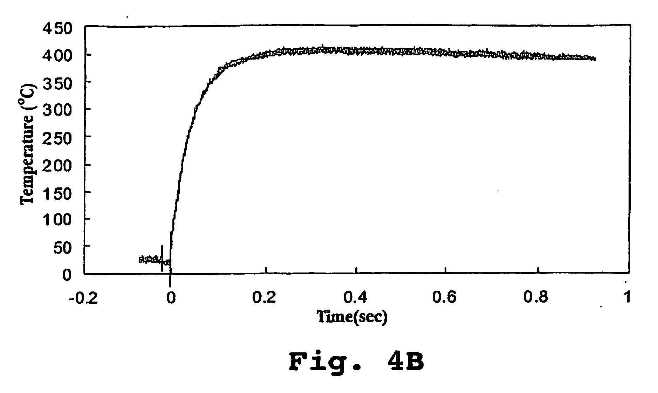

Fig. 4B shows the time-temperature relationship for a 0.005 inch thick stainless steel foil substrate that was heated by a 1 Farad capacitor charged to 16 V, again measured by a thin thermocouple (Omega Model CO2-K). The substrate reached its peak temperature of 400 °C in about 200 milliseconds, and maintained that temperature for the 1 second testing period.

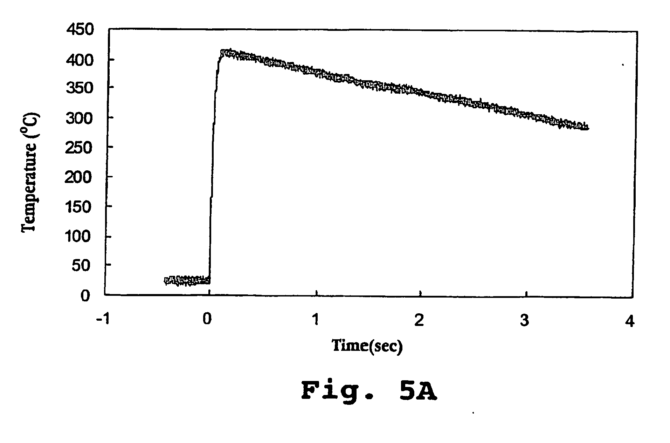

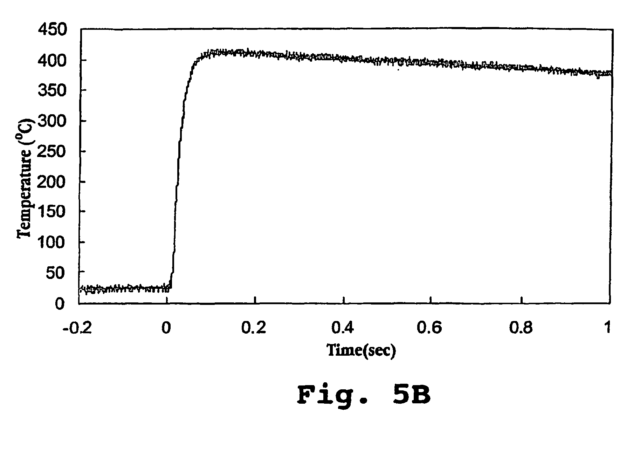

In another test, a hollow, stainless steel tube was used as the drug-film substrate. To volatilize a drug film, a cylindrical tube having a diameter of 13 mm and a length of 34 mm was connected to two I Farad capacitors wired in parallel. Figs. 5A-5B show substrate temperature measured by a thin thermocouple (Omega Model CO2-K) as a function of time, for a cylindrical substrate that had been heat-treated by heating for about three times to about 400°C for about two seconds in air. Such heat-treatment was produced by passing current through the substrate from the charged capacitors. Fig. 5B shows a detail of the first 1 second of heating.

Aluminum is another substrate material used in an aerosol generation device. The aluminum substrate in the embodiment is heated by conductive means, *e.g.*, by bringing the aluminum in contact with a heat source (*e.g.*, a halogen bulb), to vaporize the drug film. Such techniques are useful due to the higher thermal conductivity and higher electrical conductivity of aluminum relative to stainless steel. To obtain a treated exterior surface of an aluminum substrate, the substrate is placed in an oxygen-containing oven.

For each substrate type, a film comprising a drug was formed by applying a solution containing the drug onto the substrate. A variety of solvents can be used and selection is based, in part, on the solubility properties of the drug and the desired solution concentration. Common solvent choices included acetone, methanol, ethanol, acetone, chloroform, dichloromethane, other volatile organic solvents, dimethylformamide, water, and solvent mixtures. The drug solution was applied to the substrate by dip coating, yet other methods such as spray coating are contemplated as well. Alternatively, a melt of the drug can be applied to the substrate.

To determine the thickness of the film to be applied, one method that can be used is to determine the area of the substrate and calculate drug film thickness using the following relationship:

$$\text{film thickness (cm)} = \text{drug mass (g)} / [\text{drug density (g/cm}^3) \times \text{substrate area (cm}^2)]$$

The drug mass can be determined by weighing the substrate before and after formation of the drug film or by extracting the drug and measuring the amount analytically. Drug density can be experimentally determined by a variety of techniques, known by those of skill in the art or found in the literature or in reference texts, such as in the CRC. An assumption of unit density is acceptable if an actual drug density is not known.

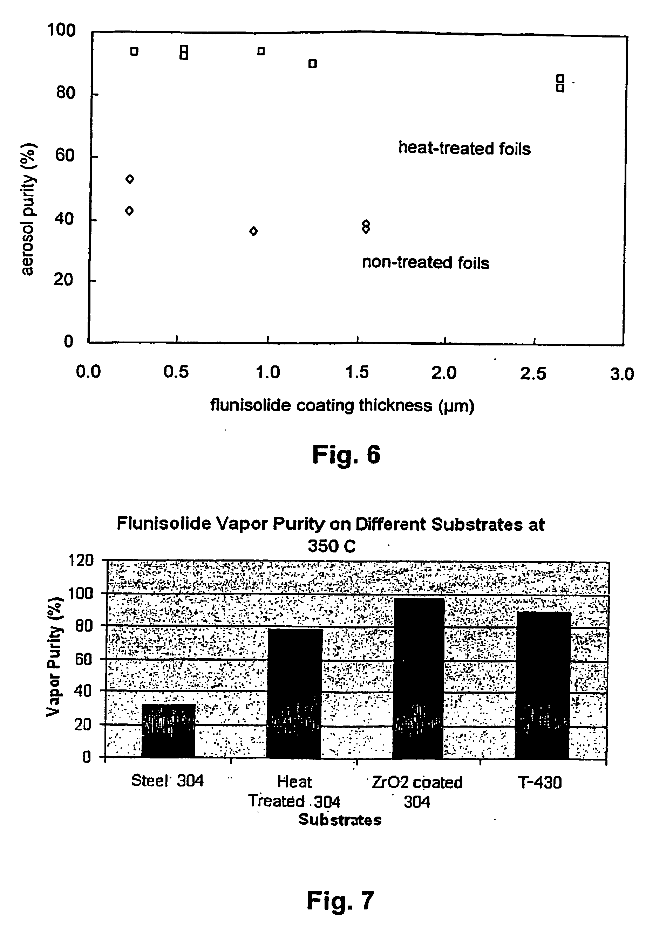

In the studies reported in the Examples, the substrate having a drug film of known thickness was heated to a temperature sufficient to generate a thermal vapor. All or a portion of the thermal vapor was recovered and analyzed for presence of drug-degradation products, to determine purity of the aerosol particles in the thermal vapor. Example 1 describes preparation of a substrate assembly containing flunisolide, a respiratory steroid used in the treatment of asthma. Prior to coating, half of the substrates were heat-treated, while another half were not heat-treated. 304 stainless steel foil substrates containing films of flunisolide ranging in thickness from between about 0.2 $\mu$m to about 2.7 $\mu$m were prepared. The coated stainless steel substrates were heated and the purity of the drug-aerosol particles in the thermal vapor generated from each substrate was determined. The results are shown in Fig. 6. Unexpectedly, there was a marked improvement in aerosol purity from the heat-treated substrates compared to the non-treated substrates. For example, for a flunisolide film having a thickness of about 0.2 $\mu$m, vaporization from a heat-treated substrate yielded a thermal vapor having a purity of about 93%; in contrast, a comparable thickness of coating from a non-treated substrate yielded a thermal vapor having a purity of only about 50%. However, surprisingly, T-430 steel foils coated with flunisolide exhibit good vapor purities without heat treatment. Further, there was no marked improvement in flunisolide aerosol purity upon vaporizing off heat-treated T-430 foils. Furthermore, flunisolide coated onto 304 stainless steel foils having an exterior surface made of zirconium oxide offers good aerosol purities without heat treatment. Similar results were also obtained at other film thicknesses for flunisolide, and also for other drugs (see Examples 2, 3, 4 and 5). The examples provided herein indicate that oxidation resistant metals or metal oxide surfaces provide improved aerosol purities compared to oxidation prone metallic substrates.

In addition to there being a relationship between substrate surface and aerosol purity, there is also a relationship between film thickness and aerosol particle purity, such that as the film thickness decreases, the purity increases. Such a relationship was found from both heat-treated and non-treated substrates. For example, from a heat-treated substrate, a flunisolide film having a thickness of about 0.2 or 0.5 $\mu$m produced a thermal vapor having a purity of about 93%; a flunisolide film having a thickness of about 2.6 $\mu$m produced a thermal vapor having a purity of 83%. From a non-treated substrate, a flunisolide film having a thickness of about 0.2 $\mu$m produce a thermal vapor having a purity of about 50%; a flunisolide film having a thickness of about 1.5 $\mu$m produced a thermal vapor having a purity of 38%.

Substrate heat-treatment also improved aerosol yield. For example, the 0.5 $\mu$m coating on the heat-treated substrate yielded about 0.3 mg of aerosol particles. This corresponds to 97% - 100% of the coated dose being emitted from the test apparatus containing the assembly comprising the flunisolide-coated, heat-treated substrate. In contrast, the 1.5 $\mu$m coating on the non-treated substrate yielded only 0.2 mg of aerosol particle, which corresponds to about 25% of the coated dose being emitted from the test apparatus. This represents not only a lower percent emitted dose for the untreated surface, but also a lower quantity of material emitted overall than that from the much thinner coating on a heat treated surface.

Thus, the nature of the substrate exterior in contact with the drug substance as well as the thickness of the drug film has an effect on aerosol production. Oxidation resistant or heat-treated substrates are effective substrates, as are other substrates with metal-oxide enriched exteriors such as, for example, chemically treated surfaces, protective overcoats, for the production of both pure aerosol and high aerosol yields.

Another feature of the drug-supply assembly is that a substrate's surface area should be sufficient to yield a therapeutic dose of the drug aerosol when used by a subject. For an aerosol delivery device or assembly of the invention, the unit dose yield may be determined by collecting the thermal vapor evolved upon actuation of the device or assembly and analyzing its composition as described herein, and comparing the results of analysis of the thermal vapor to those of a series of reference standards containing known amounts of the drug..The amount of drug or drugs required in the starting composition for delivery as a thermal vapor depends on the amount of drug or drugs entering the thermal vapor phase when heated (*i.e.,* the dose produced by the starting drug or drugs), the bioavailability of the thermal vapor phase drug or drugs, the volume of inhalation, and the potency of the thermal vapor drug or drugs as a function of plasma drug concentration.

Typically, the bioavailability of thermal vapors ranges from 20-100% and is typically in the range of 50 -100% relative to the bioavailability of drugs infused intravenously. The potency of the thermal vapor drug or drugs per unit plasma drug concentration is equal to or greater than that of the drug or drugs delivered by other routes of administration. It may substantially exceed that of oral, intramuscular, or other routes of administration in cases where the clinical effect is related to the rate of rise in plasma drug concentration more strongly than the absolute plasma drug concentration. In some instances, thermal vapor delivery results in increased drug concentration in a target organ such as the brain, relative to the plasma drug concentration (Lichtman et al., The Journal of Pharmacology and Experimental Therapeutics 279:69-76 (1996)). Thus, for medications currently given orally, the human dose or effective therapeutic amount of that drug in thermal vapor form is generally less than the standard oral dose (*e.g.,* less than 80%, more typically less than 40%, and most commonly less than 20% of the standard oral dose). For medications currently given intravenously, the drug dose in a thermal vapor will generally be similar to or less than the standard intravenous dose (*e.g.,* less than 200%, typically less than 100%, and most commonly less than 50% of the standard intravenous dose).

Determination of the appropriate dose of thermal vapor to be used to treat a particular condition can be performed via animal experiments and a dose-finding (Phase I/II) clinical trial. Such animal experiments involve measuring plasma drug concentrations after exposure of the test animal to the drug thermal vapor. These experiments may also be used to evaluate possible pulmonary toxicity of the thermal vapor. Because accurate extrapolation of these results to humans is facilitated if the test animal has a respiratory system similar to humans, mammals such as dogs or primates are typically used as the test animals. Conducting such experiments in mammals also allows for monitoring of behavioral or physiological responses in mammals. Initial dose levels for humans will generally be less than or equal to: current standard intravenous dose, current standard oral dose, dose at which a physiological or behavioral response was obtained in the mammal experiments, and dose in the mammal model which resulted in plasma drug levels associated with a therapeutic effect of drug in humans. Dose escalation may then be performed in humans, until either an optimal therapeutic response is obtained or dose-limiting toxicity is encountered.

The actual effective amount of drug for a particular subject can vary according to (i) the specific drug or combination thereof being utilized, (ii) the particular composition formulated, (iii) the mode of administration and the age, weight, and condition of the subject, and (iv) severity of the episode being treated. The amount of drug to provide a therapeutic dose is generally known in the art or can be determined as discussed above.

The dosage and the film thickness (to yield the desired aerosol purity, per the film thickness-purity relationship described herein) determine the minimum substrate area sufficient to yield a therapeutic dose of the drug aerosol when used by a subject in accord with the following relationship: film thickness (cm) x drug density (g/cm$^3$) x substrate area (cm$^2$) = dose (g)

As noted herein, drug density can be determined experimentally or from the literature, or if unknown, can be assumed to be 1 g/cc. To prepare a drug delivery device assembly comprised of a drug film on a heat-conductive substrate that is capable of administering an effective therapeutic dose, the minimum substrate surface area is determined using the relationships described above. For example, for flunisolide a film thickness of 0.5 $\mu$m at unit density coated onto 6 cm$^2$ gives a dose delivery of 0.3 mg, a therapeutic amount. Based on accommodating a therapeutic amount of compound and the desire to form an aerosol with less than 10% compound degradation via vaporization, substrates having a surface area of less than 1 mm$^2$/particle are not preferred.

The actual dose of drug delivered, i.e., the percent yield or percent emitted, from the drug-supply article will depend on, along with other factors, the percent of drug film that is vaporized upon heating the substrate. Thus, for drug films that yield 100% upon heating of the drug film and aerosol particles that have 100% drug purity, the relationship between dose, thickness, and area given above correlates directly to the dose provided to the subject. As the percent yield and/or particle purity decrease, adjustments in the substrate area can be made as needed to provide the desired dose. Also, as one of skill in the art will recognize, larger substrate areas other than the minimum calculated area for a particular film thickness can be used to deliver a therapeutically effective dose of the drug. Moreover, as can be appreciated by one of skill in art, the film need not coat the complete surface area if a selected surface area exceeds the minimum area for delivering a therapeutic dose from a selected film thickness. Although it is advantageous for all or most of the substrate exterior in contact with drug substance to be metal oxide-enriched, it is necessary for only a portion of the substrate surface to be so enriched.

As discussed above, purity of aerosol particles for many drugs correlates directly with film thickness, where thinner films typically produce aerosol particles with greater purity. Thus, one method to optimize purity disclosed is the use of thinner films. Likewise, the aerosol yield may also be optimized in this manner. Similarly, as described above, appropriate treatment of the substrate may improve aerosol purity and/or yield. There are further contemplated strategies in addition to, or in combination with, adjusting film thickness and treatment of the substrate to increase either aerosol purity or yield or both. These strategies include modifying the structure or form of the drug, and/or producing the thermal vapor in an inert atmosphere.

Thus, in one example, there is disclosed generation of and/or use of an altered form of the drug, such as, for example, use of a pro-drug, or a free base, free acid or salt form of the drug. Although not always the case, the free base or free acid form of the drug as opposed to the salt, generally results in either a higher purity or yield of the resultant aerosol. Thus, in one embodiment of the invention, the free base and free acid forms of the drugs are used.

Another approach contemplates generation of drug-aerosol particles having a desired level of drug purity by forming the thermal vapor under a controlled atmosphere of an inert gas, such as argon, nitrogen, helium, and the like.

In another example, there is disclosed, a method of forming an assembly for use in an aerosol device for producing aerosol particles of a drug composition that have the desired purity and a film that provides a desired percent yield. In one example of the method, a drug film with a known film thickness is prepared on a substrate (*e.g.*, a metal substrate). The substrate is heated to vaporize the film, thereby producing aerosol particles containing the drug compound. The purity of the aerosol particles in the thermal vapor is determined, as well as the percent yield, *i.e.*, the fraction of compound vaporized and delivered by the method. The substrate exterior surface is then optimized by use of the treatment methods described above or others known in the art to yield the desired aerosol purity. In one particular example, there is described, a method of increasing the purity of drug condensation particles in a condensation drug aerosol that is produced by substantially vaporizing and condensing a drug composition film on a substrate comprising substantially vaporizing a drug composition on an oxidation resistant or oxide-enriched metal substrate and condensing the vapor to form drug particles. For example, if a non-oxidation resistant substrate coated with a particular drug at 1 $\mu$m thickness were to yield an 80% pure aerosol, the substrate could then be heat-treated to increase the metal oxide content of the surface, coated with 0.5 $\mu$m of the drug, and the drug film could presumably vaporize to yield a 95% pure aerosol.

As can be appreciated from the above examples showing generation of a purer drug thermal vapor from thin films (*e.g.* 0.02-20 μm) of the drug coated onto a metal substrate with a treated exterior surface, the invention finds use in the medical field in compositions and articles for delivery of a therapeutic of a drug. Thus, the invention includes, in one aspect, an assembly for production of a thermal vapor that contains drug-aerosol particles. The assembly includes a treated substrate coated with a film of a drug composition to be delivered to a subject, preferably a human subject. The thickness of the drug composition film is selected such that upon vaporizing the film by heating the substrate to a temperature sufficient to vaporize at least 50% of the drug composition film, typically to a temperature of at least about 150°C to about 200°C, still more typically at least about 250°C, most commonly at least about 350°C or 400 °C, a thermal vapor is generated that has 10% or less drug-degradation product.

In another aspect, the invention relates to a method of forming a drug-supply assembly comprised of a substrate having an oxidation resistant or metal oxide-enriched exterior surface and a film comprising a drug. The metal oxide enrichment of the substrate exterior surface can be accomplished by, for example, heating or chemically treating or coating, as described above. The film of drug is then coated on the exterior surface.

A drug-supply assembly comprised of a substrate having an oxidation resistant or metal oxide-enriched exterior coated with a thin drug film is particularly suited, in another aspect of the invention, for forming a therapeutic inhalation dose of drug-aerosol particles. The inhalation route of drug administration offers several advantages for many drugs, including rapid uptake into the bloodstream, and avoidance of the first pass effect allowing for an inhalation dose of a drug that can be substantially less, *e.g.*, one half, that required for oral dosing. Efficient aerosol delivery to the lungs requires that the particles have certain penetration and settling or diffusional characteristics. For larger particles, deposition in the deep lungs occurs by gravitational settling and requires particles to have an effective settling size, defined as mass median aerodynamic diameter (MMAD), of between 1-3.5 μm. For smaller particles, deposition to the deep lung occurs by a diffusional process that requires having a particle size in the 10-100 nm, typically 20-100 nm, range. Particle sizes that fall in the range between 100 nm and I μm tend to have poor deposition and those above 3.5 μm tend to have poor penetration. Therefore, an inhalation drug-delivery device for deep lung delivery should produce an aerosol having particles in one of these two size ranges, typically between about 1-3 μm MMAD. For a drug such as flunisolide, where delivery to the small airways for the treatment of asthma is most beneficial, 1-3 μm particles are also appropriate, although slightly larger particles may also be useful.

Accordingly, a drug-supply assembly for use in an aerosol device comprising a substrate having an oxidation resistant metal oxide enriched exterior and having a drug composition film thickness selected to generate a thermal vapor having drug composition-aerosol particles with less than about 10% drug degradation product, typically less than about 5% drug degradation product, and most typically less than about 2.5 % drug degradation product, is provided. A gas, air or an inert fluid, is passed over the substrate at a flow rate effective to produce the particles having a desired MMAD. The more rapid the airflow, the more diluted the vapor and hence the smaller the particles that are formed. In other words the particle size distribution of the aerosol is determined by the concentration of the compound vapor during condensation. This vapor concentration is, in turn, determined by the extent to which airflow over the surface of the heating substrate dilutes the evolved vapor. Thus, to achieve smaller or larger particles, the gas velocity through the condensation region of the chamber may be altered by modifying the gas-flow control valve to increase or decrease the volumetric airflow rate. For example, to produce condensation particles in the size range 1-3.5 μm MMAD, the chamber may have substantially smooth-surfaced walls, and the selected gas-flow rate may be in the range of 4-50 L/minute.

Additionally, as will be appreciated by one of skill in the art, particle size may be also altered by modifying the cross-section of the chamber condensation region to increase or decrease linear gas velocity for a given volumetric flow rate, and/or the presence or absence of structures that produce turbulence within the chamber. Thus, for example to produce condensation particles in the size range 20-100 nm MMAD, the chamber may provide gas-flow barriers for creating air turbulence within the condensation chamber. These barriers are typically placed within a few thousands of an inch from the substrate surface. Typically, the flow rate of gas over the substrate ranges from about 4-50 L/min, typically from about 5-30 L/min.

Prior to, simultaneous with, or subsequent to passing a gas over the substrate, heat is applied to the substrate to vaporize the drug composition film. It will be appreciated that the temperature to which the substrate is heated will vary according to the drug's vaporization properties, but is typically heated to a temperature of at least about 150°C to at least about 200 °C, more typically at least about 250 °C, and most commonly at least about 350 °C or 400 °C. Heating the substrate produces a drug composition vapor that in the presence of the flowing gas generates aerosol particles in the desired size range. In one embodiment, the substrate is heated for a period of less than about 1 second, less than about 500 milliseconds, and still more typically for less than about 200 milliseconds. The drug-aerosol particles are inhaled by a subject for delivery to the lung.

In another embodiment, there is provided a drug-supply assembly for use in a device for producing an aerosol of drug condensation particles, *e.g.*, for use in inhalation therapy. The device has the elements described above with respect to Figs. 2A and 2B, where the heat source is designed to supply heat to the substrate in the device at a rate effective to produce a substrate temperature greater than 150 °C, or in other embodiments greater than 200 °C, 250 °C, 350 °C or 400 °C, and to substantially volatilize a drug film from the substrate in a period of 2 seconds or less. The thickness of the film of drug composition on the substrate and the treated substrate exterior surface is such that the device produces an aerosol containing less than 10% by weight drug degradation and at least 50% of the drug composition on the film.

The device includes a drug-supply assembly composed of a substrate having an oxidation resistant exterior surface (*e.g.*, a metal oxide-enriched exterior), a film of a selected drug composition on the exterior surface, and a heat source for supplying heat to the substrate at a rate effective to heat the substrate to a temperature greater than 150 °C or in other embodiments to a temperature greater than 200 °C, 250 °C, 350 °C or 400 °C, to produce substantially complete volatilization of the drug composition within a period of 2 seconds or less.

The drug film may be one that, when vaporized from a film on an impermeable surface of a heat conductive substrate, the aerosol exhibits an increasing level of drug degradation products with increasing film thicknesses, particularly at a thickness of greater than 0.05-20 microns. For this general group of drug compositions, the film thickness on the substrate will typically be between 0.05 and 20 microns, *e.g.*, the maximum or near-maximum thickness within this range that allows formation of a particle aerosol with drug degradation less than 5%.

Alternatively, the drug composition in the assembly and device may show less than 5-10% degradation even at film thicknesses greater than 20 microns. For these compounds, a film thickness greater than 20 microns, *e.g.*, 20-50 microns, may be selected, particularly where a relatively large drug dose is desired.

The drug composition in the assembly and device may be one that, when vaporized from a film on an oxidation resistant or treated exterior of a substrate, the aerosol exhibits a desired purity and drug content, but when vaporized from a comparable film on a non-treated substrate or substrate having a reference exterior, exhibits a reduced purity or drug content. In particular, the assembly may be such that, when a drug composition film is vaporized and condensed to form aerosol particles, under selected conditions that lead to at least 50% recovery of drug composition in the aerosol, the aerosol produced exhibits (i) less than about 10% by weight drug degradation products and (ii) decreased levels of drug degradation products as compared to an aerosol produced when the substrate is not a metal oxide-enriched substrate surface.

The assembly is useful in a method for producing a condensation aerosol by the steps of heating the substrate that has been heat-treated or that has a metal oxide-enriched exterior at a rate that heats the substrate to a temperature greater than 150 °C, or in other alternatives to a temperature greater than 200 °C, 250 °C, 350 °C, or 400 °C, and produces substantially complete volatilization of the compounds within a period of 2 seconds or less.

The following examples further illustrate the invention described herein and are in no way intended to limit the scope of the invention.

## Examples

Materials

Solvents were of reagent grade or better and purchased commercially.

Unless stated otherwise, the drug free base or free acid form was used in the

Examples.

Methods

A. Preparation of Drug-Coating Solution

Drug was dissolved in an appropriate solvent. Common solvent choices included methanol, dichloromethane, methyl ethyl ketone, diethyl ether, 3:1 chloroform:methanol mixture, 1:1 dichloromethane: methyl ethyl ketone mixture, dimethylformamide, and deionized water. Sonication and/or heat were used as necessary to dissolve the compound. The drug concentration was typically between 50-200 mg/mL.

B. Preparation of Drug-Coated Stainless Steel Foil Substrate

Strips of clean stainless steel foil of type 304 (0.0125 cm thick, Thin Metal Sales), type 430 (0.0124 cm thick, AK steels), type 304 coated with 1.5 micron thick zirconium oxide (vapor deposited by thin film research corporation) having dimensions 1.3 cm by 7.0 cm were dip-coated with a drug solution. The foil was then partially dipped three times into solvent to rinse drug off of the last 2-3 cm of the dipped end of the foil. Alternatively, the drug coating from this area was carefully scraped off with a razor blade. The final coated area was between 2.0-2.5 cm by 1.3 cm on both sides of the foil, for a total area of between 5.2-6.5 cm$^2$. Foils were prepared as stated above and then some were extracted with methanol or acetonitrile as standards. The amount of drug was determined from quantitative HPLC analysis. Using the known drug-coated surface area, the thickness was then obtained by:

$$\text{film thickness (cm)} = \text{drug mass (g)} / [\text{drug density (g/cm}^3\text{)} \times \text{substrate area (cm}^2\text{)}.$$

If the drug density is not known, a value of 1 g/cm$^3$ is assumed. The film thickness in microns is obtained by multiplying the film thickness in cm by 10,000.

After drying, the drug-coated foil was placed into a volatilization chamber constructed of a Delrin® block (the airway) and brass bars, which served as electrodes. The dimensions of the airway were 1.3 cm high by 2.6 cm wide by 8.9 cm long. The drug-coated foil was placed into the volatilization chamber such that the drug-coated section was between the two sets of electrodes. After securing the top of the volatilization chamber, the electrodes were connected to a 1 Farad capacitor (Phoenix Gold). The back of the volatilization chamber was connected to a two micron Teflon® filter (Savillex) and filter housing, which were in turn connected to the house vacuum. Sufficient airflow was initiated (typically 30 L/min = 1.5 m/sec), at which point the capacitor was charged with a power supply, typically to between 14-17 Volts. The circuit was closed with a switch, causing the drug-coated foil to resistively heat to temperatures of about 280-430 °C (as measured with an infrared camera (FLIR Thermacam SC3000)), in about 200 milliseconds. (For comparison purposes, see Fig. 4A, thermocouple measurement in still air.) After the drug had vaporized, airflow was stopped and the Teflon® filter was extracted with acetonitrile. Drug extracted from the filter was analyzed generally by HPLC UV absorbance generally at 225 nm using a gradient method aimed at detection of impurities to determine percent purity. Also, the extracted drug was quantified to determine a percent yield, based on the mass of drug initially coated onto the substrate. A percent recovery was determined by quantifying any drug remaining on the substrate, adding this to the quantity of drug recovered in the filter and comparing it to the mass of drug initially coated onto the substrate.

C. Preparation of Drug-Coated Stainless Steel Cylindrical Substrate

A hollow stainless steel cylinder with thin walls, typically 0.12 mm wall thickness, a diameter of 13 mm, and a length of 34 mm was cleaned in dichloromethane, methanol, and acetone, then dried, and fired at least once to remove any residual volatile material and to heat-treat the stainless steel surface. The substrate was then dip-coated with a drug coating solution. The dip-coating was done using a computerized dip-coating machine to produce a thin layer of drug on the outside of the substrate surface. The substrate was lowered into the drug solution and then removed from the solvent at a rate of typically 5-25 cm/sec. (To coat larger amounts of material on the substrate, the substrate was removed more rapidly from the solvent or the solution used was more concentrated.) The substrate was then allowed to dry for 30 minutes inside a fume hood. If either dimethylformamide (DMF) or a water mixture was used as a dip-coating solvent, the substrate was vacuum dried inside a desiccator for a minimum of one hour. The drug-coated portion of the cylinder generally has a surface area of 8 cm$^2$. By assuming a unit density for the drug, the initial drug coating thickness was calculated. The amount of drug coated onto the substrates was determined in the same manner as that described herein: the substrates were coated, then extracted with methanol or acetonitrile and analyzed with quantitative HPLC methods, to determine the mass of drug coated onto the substrate.

The drug-coated substrate was placed in a surrounding glass tube connected at the exit end via Tygon® tubing to a filter holder fitted with a Teflon® filter (Savillex). The junction of the tubing and the filter was sealed with paraffin film. The substrate was placed in a fitting for connection to two 1 Farad capacitors wired in parallel and controlled by a high current relay. The capacitors were charged by a separate power source to about 18-22 Volts and most of the power was channeled to the substrate by closing a switch and allowing the capacitors to discharge into the substrate. The substrate was heated to a temperature of between about 300-500 °C (see Fig. 5A & 5B) in about 100 milliseconds. The heating process was done under an airflow of 15 L/min, which swept the vaporized drug aerosol into a 2 micron Teflon® filter.

After volatilization, the aerosol captured on the filter was recovered for quantification and analysis. The quantity of material recovered in the filter was used to determine a percent yield, based on the mass of drug coated onto the substrate. The material recovered in the filter was also analyzed generally by HPLC UV absorbance at typically 225 nm using a gradient method aimed at detection of impurities, to determine purity of the thermal vapor. Any material deposited on the glass sleeve or remaining on the substrate was also recovered and quantified to determine a percent total recovery (((mass of drug in filter + mass of drug remaining on substrate and glass sleeve)/mass of drug coated onto substrate) x 100). For compounds without UV absorption GC/MS or LC/MS was used to determine purity and to quantify the recovery. Some samples were further analyzed by LC/MS to confirm the molecular weight of the drug and any degradants.

D. Heat Treatment of Stainless Steel Foil Substrate

Stainless steel foils (304 and T-430) were cleaned in organic solvent (such as dichloromethane, acetone, or acetonitrile) and then heated in an oven at 350 °C for 6 hours (in air). The appearance of the foils was noticeably changed, turning from silver to a bronze color.

E. Heat Treatment of Stainless Steel Cylindrical Substrate

Stainless steel cylinder substrates were cleaned in organic solvent (such as dichloromethane, acetone, or acetonitrile) and then heated to between 300°C and 500°C for approximately 5 seconds by passing electrical current through the uncoated substrate from a capacitor (as described for a drug-coated substrate above) in air. The heat treatment was repeated 1 to 5 times.

F. Base Treatment of Stainless Steel Foil Substrate

Stainless steel foils were soaked in 1N sodium hydroxide solution for 30 minutes, sonicated for 5 minutes and thoroughly washed with de-ionized water. Further, the base-treated foils were sonicated in ethanol for three to five minutes and washed with de-ionized water followed by acetone and dried at 50°C.

G. Citric Acid Treatment of Stainless Steel Foil Substrate

7.0 g of CitriSurf 2250 solution (Stellar Solutions) was diluted fivefold by adding 35 g of de-ionized water. Base-cleaned stainless steel foils were suspended in hot CitriSurf solution (90°C) for 15 minutes and then sonicated. Finally, the surface-treated foils were thoroughly washed with de-ionized water and air-dried. Alternatively, 2.0 g of citric acid (Aldrich) was dissolved in 38.0 g of distilled deionized water. Base-cleaned steel foils were suspended in hot citric acid solution (90°C) for 15 minutes and then sonicated. Finally, the foils were thoroughly washed with de-ionized water and air dried.

H. Phosphoric Acid Treatment of Stainless Steel Foil Substrate

4.0 g of 85% orthophosphoric acid was diluted by adding 36.0 g of de-ionized water. Base-cleaned steel foils were suspended in hot phosphoric acid solution (90°C) for 15 minutes and then sonicated. Finally, the treated foils were thoroughly washed with de-ionized water and air-dried.

I. Pickling of Stainless Steel Foil Substrate

A pickling solution containing 20% nitric acid and 5% hydrofluoric acid was prepared by adding 14.3 ml of 70% $HNO_3$ and 5.2 ml of 48% HF to 30.5 ml of de-ionized water. Base-cleaned stainless steel foils were suspended in the pickling solution for 3 minutes and sonicated for a minute (the pickling solution turns slightly green) and then thoroughly washed with de-ionized water. A batch of pickled stainless steel foil samples were additionally treated with 1N NaOH and then washed with water, while the other batch of samples was washed with acetone. Both of them were dried at 100°C.

J. Nitric Acid Treatment of Stainless Steel Foil Substrate

4.0 g of 70% nitric acid was diluted by adding 36 g of de-ionized water. Base-cleaned steel foils were suspended in diluted nitric acid solution and sonicated for five minutes. The stainless steel foils were rinsed with acetone and air dried after thorough washing with deionized water.

K. Zirconium oxide overcoat on 304 Stainless Steel

1.5 micron thick zirconium oxide coating was deposited onto clean surface of 0.0125 cm thick 304 stainless steel foils using ion assisted physical vapor deposition process at the research facility of Thin Films Research Incorporation, Westford, MA.

**Example 1**

**Generation of flunisolide aerosol from clean and treated stainless steel substrates 304 and T-430:** Strips of steel foils 304 (0.0125 cm thick, Thin Metal Sales), T-430 (0.0125 cm thick, AK steels), and 304 foils coated with zirconium oxide (0.0125 cm thick, coated with 1.5 micron thick ZrO$_2$ overcoat, Thin films Research Inc.) having dimensions 1.3 cm by 7.0 cm were cleaned by sonication in 6.5% Ridoline 298 aqueous solution for 30 min followed by thorough rinsing with DI water and acetone. Half of non-zirconium oxide coated 304 foil and half of the T-430 steel foils were heated in an oven at 350 °C for 6 hours in an air atmosphere. As a result of the heating, these foils were oxidized, and underwent a color change from silver to bronze. All foils were dip-coated with a flunisolide solution in dichloromethane. The concentration of the solution was varied to alter the flunisolide coating thickness on the steel foils. After drying, the drug coating from the last 2-3 cm was carefully scraped off with a razor blade. Foils were subsequently vaporized as described in herein. The discharge voltage was set to 13.5V for 304 steel foils, 14.5 Volts for T-430 and 14.0 Volts for 304 coated with zirconium oxide to achieve peak substrate temperature of about 350 °C, as measured by an infrared camera (FLIR Thermacam SC3000).

In all cases, the quantity of drug remaining on the foil after vaporization was less than 15% of the loaded dose.

For the heat-treated substrate 304 having a drug film thickness of 0.5 $\mu$m, 0.253 mg of drug was applied to the substrate. After volatilization of drug from this substrate, 0.253 mg was recovered from the filter, for a percent yield of 100%. Purity of the drug aerosol particles was 94.4%. A total mass of 0.253 mg was recovered from the test apparatus and substrate, for a total recovery of 100%.

For the non-treated substrate 304 having a drug film thickness of 0.9 $\mu$m, 0.485 mg of drug was applied to the substrate. After volatilization of drug from this substrate, 0.206 mg was recovered from the filter, for a percent yield of 42.4%. Purity of the drug aerosol particles was 36.3%. A total mass of 0.210 mg was recovered from the test apparatus and substrate, for a total recovery of 43.3%.

For the non-treated substrate T-430 having a drug film thickness of 1.3 $\mu$m, 0.68 mg of drug was applied to the substrate. After volatilization of drug from this substrate, 0.47 mg was recovered from the filter, for a percent yield of 69.1 %. Purity of the drug aerosol particles was 82.8%. A total mass of 0.51 mg was recovered from the test apparatus and substrate, for a total recovery of 75.0%.

For the substrate 304 having a drug film thickness of 1.2 $\mu$m, 0.65 mg of drug was applied to the substrate. After volatilization of drug from this substrate, 0.161 mg was recovered from the filter, for a percent yield of 24.7%. Purity of the drug aerosol particles was 30.2%. A total mass of 0.172 mg was recovered from the test apparatus and substrate, for a total recovery of 26.4 %.

For the heat-treated substrate 304 having a drug film thickness of 1.5 $\mu$m, 0.443 mg of drug was applied to the substrate. After volatilization of drug from this substrate, 0.255 mg was recovered from the filter, for a percent yield of 57.5%. Purity of the drug aerosol particles was 76.5%. A total mass of 0.265 mg was recovered from the test apparatus and substrate, for a total recovery of 59.9%.

For the zirconium oxide coated substrate 304 having a drug film thickness of 0.8 $\mu$m, 0.44 mg of drug was applied to the substrate. After volatilization of drug from this substrate, 0.246 mg was recovered from the filter, for a percent yield of 55.9%. Purity of the drug aerosol particles was 90.0%. A total mass of 0.31 mg was recovered from the test apparatus and substrate, for a total recovery of 70.0%.

The thermal drug vapor purity formed upon heating of flunisolide films of between 0.2 $\mu$m and 2.6 $\mu$m from heat-treated versus non-treated stainless steel substrates 304 is shown in Fig. 6. The heat-treated foils show a marked increase in the flunisolide aerosol purity. In addition, as noted above, non-treated T-430 foils and 304 foils coated with zirconium oxide also provide improved purities compared to non-treated 304 foils, see Fig. 7.

**Example 2**

<u>Generation of eletriptan aerosol from heat-treated stainless steel substrate having a heat-treated exterior:</u> Strips of 304 stainless-steel foil (0.0125 cm thick, Thin Metal Sales) having dimensions 1.3 cm by 7.0 cm were cleaned by sonication in 6.5% Ridoline 298 aqueous solution for 30 min followed by thorough rinsing with DI water and acetone. Half of the foils were heated in an oven at 350 °C for 6 hours with air flow into the oven. As a result of the heating, these foils became strongly oxidized, and underwent a color change from silver to bronze. All foils were dip-coated with an eletriptan solution in acetone. The concentration of the solution was varied to alter the eletriptan coating thickness on the steel foils. Foils were subsequently vaporized as described in herein. The discharge voltage was set to 17.5 Volts, which results in a peak substrate temperature of about 450 °C, as measured by an infrared camera (FLIR Thermacam SC3000).

In all cases, the quantity of drug remaining on the foil after vaporization was less than 15% of the loaded dose.

For the heat-treated substrate having a drug film thickness of 4.2 $\mu$m, 2.26 mg of drug was applied to the substrate. After volatilization of drug from this substrate, 1.78 mg was recovered from the filter, for a percent yield of 78.9%. Purity of the drug aerosol particles was 95.6%. A total mass of 1.79 mg was recovered from the filter, test apparatus, and substrate, for a total recovery of 79.3%.

For the non-treated substrate having a drug film thickness of 4.2 $\mu$m, 2.26 mg of drug was applied to the substrate. After volatilization of drug from this substrate, 1.647 mg was recovered from the filter, for a percent yield of 72.9%. Purity of the drug aerosol particles was 92.8%. A total mass of 1.65 mg was recovered from the filter, test apparatus and substrate, for a total recovery of 73%.

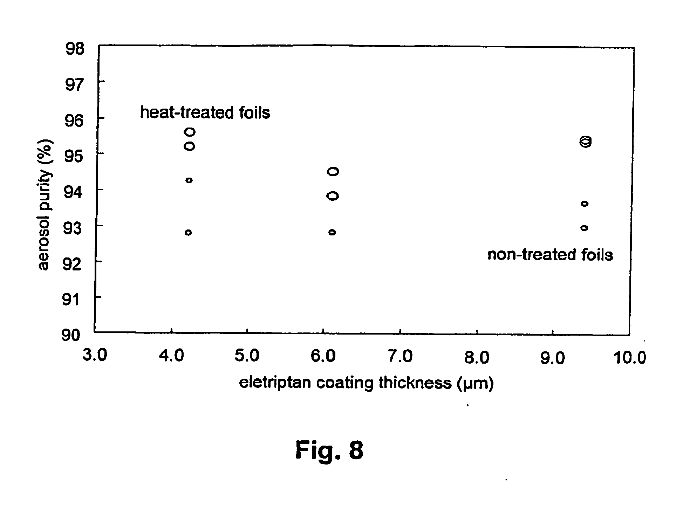

The thermal drug vapor purity between 4.0 and 9.5 $\mu$m is shown in Fig. 8. The heat-treated foils show an increase in the eletriptan aerosol purity.

## Example 3

### Generation of alprazolam aerosol from heat-treated stainless steel substrate:

Strips of 302/304 stainless-steel foil (0.00125 cm thick, Thin Metal Sales), having dimensions 6.8 cm by 1.3 cm, were cleaned by rinsing with dichloromethane. One-third of the foils were then heated in an oven at 350 °C for 1 hour. Another third of the foils were heated in an oven at 350 °C for 6 hours. As a result of the heating, these foils became strongly oxidized, and underwent a color change from silver to bronze. All foils were dip-coated with an alprazolam solution in dichloromethane. The concentration of the solution was 50 mg/mL. The foil was then partially dipped two times into pure dichloromethane to rinse drug off of the bottom of the dipped end of the foil. The final coated area was about 2 cm by 1.3 cm on both sides of the foil, for a total area of about 5.2 cm$^2$. Several foils, of both the control and the two heat-treated groups, were extracted immediately with acetonitrile and quantified on HPLC. These amounts were used to determine the loaded dose of alprazolam, which in conjunction with the known coated surface area, allowed us to calculate the drug coating thickness. There was no significant difference in the amount of alprazolam coated on the different foil lots. The coating thickness was calculated to be 0.8-1.0 $\mu$m.

After drying, the drug-coated foil was placed into a volatilization chamber constructed of a Delrin® block (the airway) and brass bars, which served as electrodes. The dimensions of the airway were 1.3 high by 2.6 wide by 8.9 cm long. The drug-coated foil was placed into the volatilization chamber such that the drug-coated section was between the two sets of electrodes. After securing the top of the volatilization chamber, the electrodes were connected to a 1 Farad capacitor (Phoenix Gold). The back of the volatilization chamber was connected to a glass fiber filter (Pall Gelman) and filter housing, which were in turn connected to the house vacuum. Airflow (45 L/min) was initiated, at which point the capacitor was charged with a power supply to 7.0 Volts. The circuit was closed with a switch, causing the drug-coated foil to resistively heat. After the drug had vaporized, airflow was stopped and the filter was extracted with acetonitrile. Drug extracted from the filter was analyzed by HPLC UV absorbance at 225 nm using a gradient method aimed at detection of impurities to determine percent purity. In all cases, the quantity of drug remaining on the foil after vaporization was less than 10% of the loaded dose.

For the non-treated substrate, 0.419 mg of drug was applied to the substrate. After volatilization of drug from this substrate, 0.405 mg was recovered from the filter, for a percent yield of 96.8%. Purity of the drug aerosol particles was 98.4%. A total mass of 0.405 mg was recovered from the filter, test apparatus, and substrate, for a total recovery of 96.8%.

For the I hour heat-treated substrate, 0.442 mg of drug was applied to the substrate. After volatilization of drug from this substrate, 0.422 mg was recovered from the filter, for a percent yield of 95.7%. Purity of the drug aerosol particles was 99.5%. A total mass of 0.427 mg was recovered from the filter, test apparatus, and substrate, for a total recovery of 96.5%.

For the 6 hour heat-treated substrate, 0.519 mg of drug was applied to the substrate. After volatilization of drug from this substrate, 0.516 mg was recovered from the filter, for a percent yield of 99.4%. Purity of the drug aerosol particles was 99.6%. Total drug recovered from the filter, test apparatus, and substrate was ~100%.

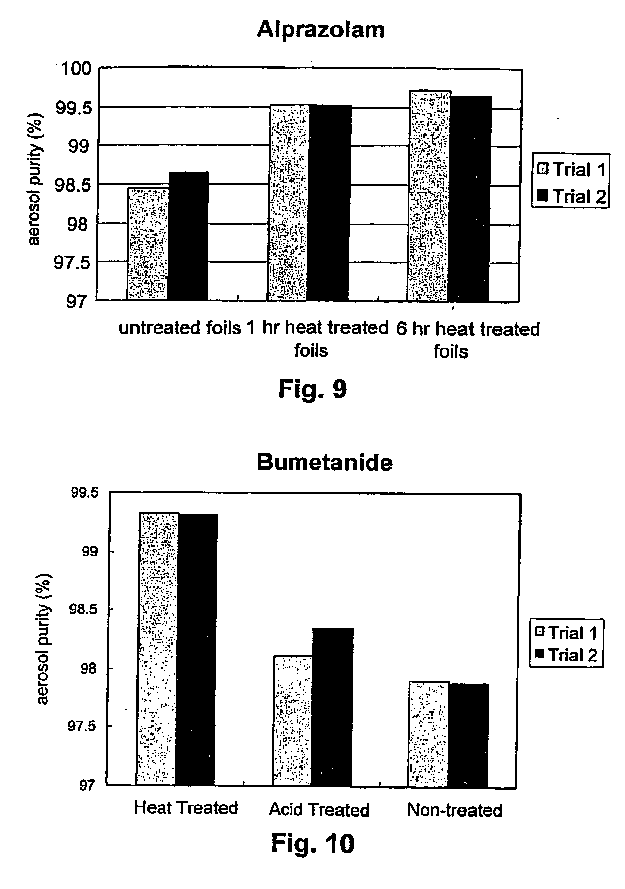

Fig. 9 shows the purities of two experiments for each of the three conditions. Note that drug vapor purity increases from using heat-treated stainless steel substrates.

## Example 4

**Generation of bumetanide aerosol from heat-treated and acid-treated stainless steel substrates having a metal oxide-enriched exterior:** Strips of 304 stainless-steel foil (0.0125 cm thick, Thin Metal Sales) having dimensions 1.3 cm by 7.0 cm were cleaned by sonication in 6.5% Ridoline 298 aqueous solution for 30 min followed by thorough rinsing with DI water and acetone. One-third of the foils were heated in an oven at 350 °C for 6 hours in an air atmosphere. As a result of the heating, these foils became strongly oxidized, and underwent a color change from silver to bronze. Another one-third of the foils were treated by washing in nitric acid, following procedure J except using a sonication time of 30 minutes. All foils were dip-coated with a bumetanide solution in 5:1 methanol:dichloromethane. The drug coating from the last few cm was carefully scraped off with a razor blade. The final coated area was about 2.2 cm by 1.3 cm on both sides of the foil, for a total area of about S.5 cm$^2$. Several foils, of the control, acid-treated, and heat-treated groups, were extracted with acetonitrile and quantified on HPLC. These amounts determined the average loaded dose of bumetanide to be 0.77 mg. There was no significant difference in the amount of bumetanide coated on the different foil lots. In conjunction with the known coated surface area, this allowed us to calculate the average drug coating thickness of 1.4μm. Foils were subsequently vaporized as described in herein. The discharge voltage was set to 15.0 Volts, which results in a peak substrate temperature of about 320 °C, as measured by an infrared camera (FLIR Thermacam SC3000).

In all cases, the quantity of drug remaining on the foil after vaporization was less than 5% of the loaded dose.

For the heat-treated substrate, 0.77 mg of drug was applied to the substrate. After volatilization of drug from this substrate, 0.40 mg was recovered from the filter, for a percent yield of 52%. Purity of the drug aerosol particles was 99.3%. A total mass of 0.41 mg was recovered from the filter, test apparatus, and substrate, for a total recovery of 53%.

For the acid-treated substrate, 0.77 mg of drug was applied to the substrate. After volatilization of drug from this substrate, 0.41 mg was recovered from the filter, for a percent yield of 53%. Purity of the drug aerosol particles was 98.3%. A total mass of 0.42 mg was recovered from the filter, test apparatus and substrate, for a total recovery of 55%.

For the non-treated substrate, 0.77 mg of drug was applied to the substrate. After volatilization of drug from this substrate, 0.37 mg was recovered from the filter, for a percent yield of 48%. Purity of the drug aerosol particles was 97.9%. A total mass of 0.37 mg was recovered from the filter, test apparatus and substrate, for a total recovery of 48%.

**Example 5**

**Generation of budesonide aerosol from clean and treated steel foils 304 and T-430.** Strips of steel foils 304 (0.0125 cm thick, Thin Metal Sales), T-430 (0.0125 cm thick, AK steels), 304 foils coated with zirconium oxide (0.0125 cm thick coated with 1.5 micron thick $ZrO_2$ overcoat, Thin films Research Inc.) having dimensions 1.3 cm by 7.0 cm were cleaned by sonication in 6.5% Ridoline 298 aqueous solution for 30 min followed by thorough rinsing with DI water and acetone. Half of 304 and T-430 steel foils were heated in an oven at 350 °C for 6 hours in an air atmosphere. As a result of the heating, these foils became oxidized, and underwent a color change from silver to bronze. All foils were dip-coated with a budesonide solution in dichloromethane. The drug coating from the last few cm was carefully scraped off with a razor blade. The final coated area was about 2.2-2.5 cm by 1.3 cm on both sides of the foil, for a total area of about 5.7-6.5 cm$^2$. Several foils, of the control were extracted with acetonitrile and quantified on HPLC. These amounts determined the average loaded dose of budesonide to be 0.55 mg (clean and heat treated 304, clean T-430), 0.85 mg (heat treated T-430), and 0.28 mg (304 coated with zirconium oxide). In conjunction with the known coated surface area, this allowed us to calculate the average drug coating thicknesses. Foils were subsequently vaporized as described in herein. The discharge voltage was set to 13.5V for 304 steel foils, 14.5 Volts for T-430 and 14.0 Volts for 304 coated with zirconium oxide to achieve peak substrate temperature of about 350 °C, as measured by an infrared camera (FLIR Thermacam SC3000)

In all cases, the quantity of drug remaining on the foil after vaporization was less than 5% of the loaded dose.

For the clean substrate 304, 0.55 mg of drug was applied to the substrate. After volatilization of drug from this substrate, 0.067 mg was recovered from the filter, for a percent yield of 12.7%. Purity of the drug aerosol particles was 23.1%. A negligible amount of budesonide was recovered from the test apparatus, and substrate.

For the clean substrate T-430, 0.55 mg of drug was applied to the substrate. After volatilization of drug from this substrate, 0.37 mg was recovered from the filter, for a percent yield of 68.5%. Purity of the drug aerosol particles was 73.3%. A total mass of 0.44 mg was recovered from the filter, test apparatus and substrate, for a total recovery of 80%.

For the heat treated substrate 304, 0.60 mg of drug was applied to the substrate. After volatilization of drug from this substrate, 0.373 mg was recovered from the filter, for a percent yield of 62%. Purity of the drug aerosol particles was 71.6%. A total mass of 0.373 mg was recovered from the filter, test apparatus and substrate, for a total recovery of 62%.

For the heat treated substrate T-430, 0.85 mg of drug was applied to the substrate. After volatilization of drug from this substrate, 0.47 mg was recovered from the filter, for a percent yield of 55.5%. Purity of the drug aerosol particles was 66.9.9%. A total mass of 0.472 mg was recovered from the filter, test apparatus and substrate, for a total recovery of 55.5%.

For the 304 substrate coated with zirconium oxide, 0.28 mg of drug was applied to the substrate. After volatilization of drug from this substrate, 0.162 mg was recovered from the filter, for a percent yield of 57.8%. Purity of the drug aerosol particles was 81.8%. A total mass of 0.176 mg was recovered from the filter, test apparatus and substrate, for a total recovery of 62.8%.

**Example 6**

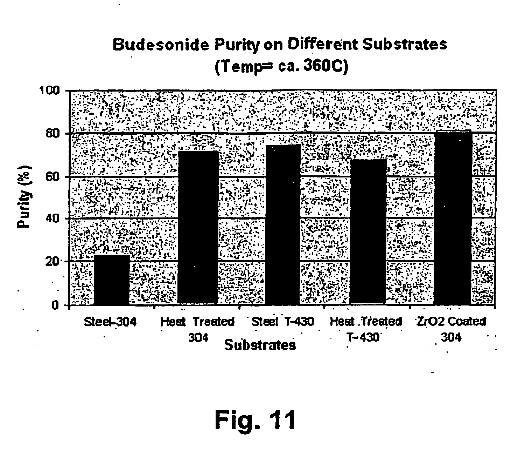

**X-ray Photoelectron Spectroscopy Studies on Non-Treated and Heat Treated 304 and T-430 Foils.** XPS analysis was carried out (at Charles Evans & Associates of Sunnyvale, CA) on non-treated and heat-treated (in air at 350°C for 6 h) steel foils 304 and T-430 to determine the surface elemental composition of the substrates. Because the chemical vapor purity of the drugs is deemed to be in direct correlation with the surface properties of heating substrates. XPS data is quantified using relative sensitivity factors and a model that assumes a homogeneous layer. The analysis volume is the product of the area of analysis (spot size or aperture size) and the depth of information. Photoelectrons are generated within the X-ray penetration depth (typically many microns), but only the photoelectrons within the top three photoelectron escape depths are detected. Analytical parameters of this experiment are described in Table 1. Escape depths are on the order of 15-35 Å, which leads to an analysis depth of~50-100 Å. Typically, 95% of the signal originates from within this depth. Tables 2 and 3 reveal the chemical composition of non-treated and heat treated steel foils 304 and 430. It is clear from these two tables that heat treatment increases the oxide content on surface and reduces the reactive metal content (*e.g.* Fe). Further, T-430 steel foils, which are known for their oxidation resistance at higher temperatures, have low content of pure metals or their alloys (*e.g.,* low iron and no nickel) and higher content of inert metal oxides (*e.g.* silicon oxide) on its surface. Probably, this explains the reason for improved purities observed for drugs flunisolide and budesonide over T 430 steel foils compared to 304 steel foils (see Figs. 7 and 11).

**Table 1. Analytical Parameters**

| | |
|---|---|
| Instrument | PHI Quantum 2000 |
| X-ray source | Monochromated Alk$_\alpha$ 1486.6eV |
| Acceptance Angle | $\pm23°$ |
| Take-off angle | 45° |
| Analysis area | 1400$\mu$m x 300$\mu$m |
| Charge Correction | C1s 284.8 eV |

**Table 2: Atomic Concentration of non-treated and heat treated steel foils 304 (in %)[a]**

| Sample | C | N | O | Na | Si | P | S | Cl | K | Ca | Cr | Mn | Fe | Ni |
|---|---|---|---|---|---|---|---|---|---|---|---|---|---|---|
| Non-treated 304 foils | 24.3 | 1.0 | 51.0 | 1.9 | 0.4 | 2.5 | 0.8 | -[b] | 0.1 | 1.1 | 4.8 | - | 11.5 | 0.6 |
| Heat treated 304 @350°C for 6h | 13.1 | - | 62.8 | 1.0 | 1.5 | - | 02 | 0.3 | 0.2 | - | 0.1[c] | 0.2 | 20.5 | - |

[a] Normalized to 100% of the elements detected. XPS does not detect H or He.
[b] A dash line "-" indicates the element is not detected.
[c] This is the maximum Cr possible; low levels of Cr are difficult to quantify due to interference from NaKLL lines.

**Table 3: Atomic Concentrations of non-treated and heat treated steel foils T-430 (in %)[a]**

| Sample | C | N | O | F | Na | Si | S | Cr | Ca | V | Cr | Mn | Fe |
|---|---|---|---|---|---|---|---|---|---|---|---|---|---|
| T-430-0hr | 324 | 4.1 | 39.1 | -[b] | - | 8.8 | 0.2 | - | 0.3 | 0.7 | 8.6 | 1.1 | 3.8 |
| T-430-6hr | 28.1 | 0.6 | 53.3 | 0.3 | 0.3 | 1.1 | 0.2 | 0.2 | 0.3 | 0.1 | 1.1 | 0.7 | 13.2 |

[a] Normalized to 100% of the elements detected. XPS does not detect H or He.
[b] A dash line "-" indicates the element is not detected.

The foregoing examples illustrate various aspects of the invention and practice of the manufacturing methods of the invention. The examples are not intended to provide an exhaustive description of the many different embodiments of the invention.

**Claims**

1. A drug-supply assembly (10) comprising:

   a metal substrate (12);
   a film (18) comprising a drug compound on the exterior surface (14) of the substrate (12);

   **characterized in that** the metal substrate (12) has a metal oxide-enriched exterior surface (14).

2. The drug-supply assembly of claim 1, wherein the metal is selected from the group consisting of steel, stainless steel, aluminum, chromium, copper, iron, and titanium.

3. The drug-supply assembly of claim 2, wherein the metal is stainless steel.

4. The drug-supply assembly of any one of claims 1 to 3, wherein the metal oxide-enriched exterior surface comprises an oxide layer of greater than 7.5 nm in thickness.

5. The drug-supply assembly of claim 4, wherein the metal oxide-enriched exterior surface comprises an oxide layer of greater than 10 nm in thickness.

6. The drug-supply assembly of claim 4, wherein the metal oxide-enriched exterior surface comprises an oxide layer of greater than 20 nm in thickness.

7. The drug-supply assembly of any one of claims I to 6, wherein the films (18) is about 0.05 to 20 microns ($\mu$m) thick.

8. A drug-supply assembly according to any one of claims to 7 wherein the drug-supply assembly is incorporated in an inhalation device (30) capable of producing an aerosol of said drug compound.

9. A method of making a drug-supply assembly (10) comprising:

   providing a metal substrate (12) having an exterior surface (14);
   treating the substrate (12) to result in a metal-oxide enriched exterior surface (14); and
   coating at least a portion of the metal oxide-enriched exterior surface (14) with a drug compound to generate a film (18).

10. The method of claim 9, wherein the treating is by contacting the metal substrate with a chemical that oxidizes the exterior surface.

11. The method of claim 9, wherein the treating is by heating the metal substrate to oxidize the exterior surface.

12. The method of claim 9, wherein the exterior surface is treated with an overcoat of an oxidation resistant material.

13. The method of claim 10, wherein the chemical is an acid.

14. The method of claim 10, wherein the chemical is a base.

15. The method of claim 11, wherein the heating is at 350 °C for 6 hours.

**Patentansprüche**

1. Arzneimittelzufuhranordnung (10), umfassend:

   ein Metallsubstrat (12);
   einen Film (18), der eine Arzneimittelverbindung auf der Außenfläche (14) des Substrats (12) umfasst;
   **dadurch gekennzeichnet, dass** das Metallsubstrat (12) eine mit Metalloxid angereicherte Außenfläche (14) aufweist.

2. Arzneimittelzufuhranordnung nach Anspruch 1, worin das Metall aus der aus Stahl, Edelstahl, Aluminium, Chrom, Kupfer, Eisen und Titan bestehenden Gruppe ausgewählt ist.

3. Arzneimittelzufuhranordnung nach Anspruch 2, worin das Metall Edelstahl ist.

4. Arzneimittelzufuhranordnung nach einem der Ansprüche 1 bis 3, worin die mit Metalloxid angereicherte Außenfläche eine Oxidschicht mit einer Dicke von mehr als 7,5 nm umfasst.

5. Arzneimittelzufuhranordnung nach Anspruch 4, worin die mit Metalloxid angereicherte Außenfläche eine Oxidschicht mit einer Dicke von mehr als 10 nm umfasst.

**6.** Arzneimittelzufuhranordnung nach Anspruch 4, worin die mit Metalloxid angereicherte Außenfläche eine Oxidschicht mit einer Dicke von mehr als 20 nm umfasst.

**7.** Arzneimittelzufuhranordnung nach einem der Ansprüche 1 bis 6, worin der Film (18) etwa 0,05 bis 20 μm dick ist.

**8.** Arzneimittelzufuhranordnung nach einem der Ansprüche 1 bis 7, worin die Arzneimittelzufuhranordnung in eine Inhalationsvorrichtung (30) eingebaut ist, die in der Lage ist, ein Aerosol der Arzneimittelverbindung zu produzieren.

**9.** Verfahren zur Herstellung einer Arzneimittelzufuhranordnung (10), umfassend:

das Bereitstellen eines Metallsubstrats (12) mit einer Außenfläche (14);
das Behandeln des Substrats (12), so dass es zu einer mit Metalloxid angereicherten Außenfläche (14) wird; und
das Beschichten zumindest eines Abschnitts der mit Metalloxid angereicherten Außenfläche (14) mit einer Arzneimittelverbindung, um einen Film (18) zu erzeugen.

**10.** Verfahren nach Anspruch 9, worin das Behandeln durch das Kontaktieren des Metallsubstrats mit einer Chemikalie stattfindet, die die Außenfläche oxidiert.

**11.** Verfahren nach Anspruch 9, worin das Behandeln durch das Erhitzen des Metallsubstrats zum Oxidieren der Außenfläche stattfindet.

**12.** Verfahren nach Anspruch 9, worin die Außenfläche mit einer Beschichtung eines oxidationsbeständigen Materials behandelt wird.

**13.** Verfahren nach Anspruch 10, worin die Chemikalie eine Säure ist.

**14.** Verfahren nach Anspruch 10, worin die Chemikalie eine Base ist.

**15.** Verfahren nach Anspruch 11, worin das Erhitzen 6 h lang bei 350 °C stattfindet.


**Revendications**

**1.** Ensemble d'administration de médicaments (10) comprenant:

un substrat métallique (12);
un film (18) comprenant un composé de médicament sur la surface extérieure (14) du substrat (12);
**caractérisé en ce que** le substrat métallique (12) possède une surface extérieure enrichie en oxyde métallique (14).

**2.** Ensemble d'administration de médicaments selon la revendication 1, dans lequel le métal est sélectionné dans le groupe consistant en acier, acier inoxydable, aluminium, chrome, cuivre, fer et titane.

**3.** Ensemble d'administration de médicaments selon la revendication 2, dans lequel le métal est de l'acier inoxydable.

**4.** Ensemble d'administration de médicaments selon l'une quelconque des revendications 1 à 3, dans lequel la surface extérieure enrichie en oxyde métallique comprend une couche d'oxyde d'une épaisseur supérieure à 7,5 nm.

**5.** Ensemble d'administration de médicaments selon la revendication 4, dans lequel la surface extérieure enrichie en oxyde métallique comprend une couche d'oxyde d'une épaisseur supérieure à 10 nm.

**6.** Ensemble d'administration de médicaments selon la revendication 4, dans lequel la surface extérieure enrichie en oxyde métallique comprend une couche d'oxyde d'une épaisseur supérieure 20 nm.

**7.** Ensemble d'administration de médicaments selon l'une quelconque des revendications 1 à 6, dans lequel le film (18) a une épaisseur d'environ 0,05 à 20 microns (μm).

8. Ensemble d'administration de médicaments selon l'une quelconque des revendications 1 à 7, dans lequel l'ensemble d'administration de médicaments est incorporé dans un dispositif d'inhalation (30) apte à produire un aérosol dudit composé de médicament.

9. Procédé de fabrication d'un ensemble d'administration de médicaments (10) comprenant:

   réaliser un substrat métallique (12) ayant une surface extérieure (14);
   traiter le substrat (12) pour obtenir une surface extérieure enrichie en oxyde métallique (14); et
   revêtir au moins une portion de la surface extérieure enrichie en oxyde métallique (14) d'un composé de médicament pour produire un film (18).

10. Procédé selon la revendication 9, dans lequel le traitement a lieu en mettant en contact le substrat métallique avec un agent chimique qui oxyde la surface extérieure.

11. Procédé selon la revendication 9, dans lequel le traitement a lieu en chauffant le substrat métallique pour oxyder la surface extérieure.

12. Procédé selon la revendication 9, dans lequel la surface extérieure est traitée avec une surcouche d'un matériau résistant à l'oxydation.

13. Procédé selon la revendication 10, dans lequel l'agent chimique est un acide.

14. Procédé selon la revendication 10, dans lequel l'agent chimique est une base.

15. Procédé selon la revendication 11, dans lequel le chauffage a lieu à 350°C pendant 6 heures.

Fig. 1A

Fig. 1B

Fig. 2A

Fig. 3A    $t = 0$ ms

Fig. 3B    $t = 50$ ms

Fig. 3C    $t = 100$ ms

Fig. 3D    $t = 200$ ms

Fig. 3E    $t = 500$ ms

Fig. 2B

Fig. 4A

Fig. 4B

29

Fig. 5A

Fig. 5B

**Fig. 6**

**Fig. 7**

Fig. 8

## Alprazolam

**Fig. 9**

## Bumetanide

**Fig. 10**

**Budesonide Purity on Different Substrates**
**(Temp= ca. 360C)**

**Fig. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02098389 A

- WO 2004104490 A

**Non-patent literature cited in the description**

- **Lichtman et al.** *The Journal of Pharmacology and Experimental Therapeutics,* 1996, vol. 279, 69-76